(19) Europäisches Patentamt
European Patent Office
Office européen des brevets

(11) Veröffentlichungsnummer: 0 352 575 A2

(12) EUROPÄISCHE PATENTANMELDUNG

(21) Anmeldenummer: 89113011.4

(22) Anmeldetag: 15.07.89

(51) Int. Cl.4: C07D 471/04 , C07D 487/04 , C07D 513/04 , C07D 498/04 , A61K 31/435 , A61K 31/55 , //(C07D471/04,221:00,209:00), (C07D487/04,223:00,209:00)

(30) Priorität: 28.07.88 DE 3825611
13.01.89 IT 1908489

(43) Veröffentlichungstag der Anmeldung: 31.01.90 Patentblatt 90/05

(84) Benannte Vertragsstaaten: AT BE CH DE ES FR GB GR IT LI LU NL SE

(71) Anmelder: BAYER AG
D-5090 Leverkusen 1 Bayerwerk(DE)

(72) Erfinder: Philipps, Thomas, Dr.
Silesiusstrasse 74
D-5000 Köln 80(DE)
Erfinder: Angerbauer, Rolf, Dr.
Moospfad 20
D-5600 Wuppertal(DE)
Erfinder: Fey, Peter, Dr.
Ursulastrasse 14
D-5600 Wuppertal 1(DE)
Erfinder: Hübsch, Walter, Dr.
Am Eckbusch 43/50
D-5600 Wuppertal 1(DE)
Erfinder: Bischoff, Hilmar, Dr.
Am Rohm 78
D-5600 Wuppertal 1(DE)
Erfinder: Petzinna, Dieter, Dr.
Peter-Berten-Strasse 10
D-4000 Düsseldorf 12(DE)
Erfinder: Schmidt, Delf, Dr.
Am Eckbusch 55b
D-5600 Wuppertal 1(DE)
Erfinder: Thomas, Günter, Dr.
Via Campogallo 21/14
I-20020 Arese (Milano)(IT)

(54) **Substituierte anellierte Pyrrole.**

(57) Substituierte anellierte Pyrrole können durch Reduktion entsprechender Ketone und nachfolgender Verseifung, Cyclisierung, Hydrierung und gegebenenfalls Isomerentrennung hergestellt werden. Die neuen substituierten annellierten Pyrrole können in Arzneimitteln verwendet werden.

EP 0 352 575 A2

## Substituierte anellierte Pyrrole

Die Erfindung betrifft substituierte anellierte Pyrrole, Zwischenverbindungen zu ihrer Herstellung und ihre Verwendung in Arzneimitteln.

Es ist bekannt, daß aus Pilzkulturen isolierte Lactonderivate Inhibitoren der 3-Hydroxy-3-methyl-glutaryl-Coenzym A-Reduktase (HMG-CoA-Reduktase) sind [Mevinolin, EP 22 478, US 4 231 938]. Darüberhinaus sind auch bestimmte Indolderivate bzw. Pyrazolderivate [EP-A 1 114 027; US 4 613 610] sowie Pyrrolderivate [EP-A 1 0 221 025] Inhibitoren der HMB-CoA-Reduktase.

Es wurden nun substituierte anellierte Pyrrole der allgemeinen Formel (I),

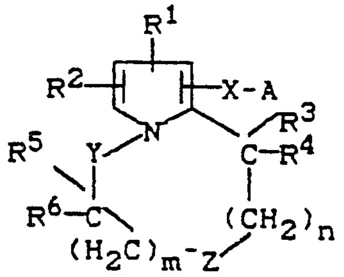

(I)

in welcher

$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$, $R^8$ - gleich oder verschieden sind und für Alkyl, Aryl, Aralkyl, Acyl, Alkylsulfonyl oder Arylsulfonyl stehen,

oder

$R^1$ - Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben,

$R^2$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$, $R^8$ - die oben angegebene Bedeutung haben,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben,

oder

- Cycloalkyl bedeutet, oder

- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,

worin

$R^7$, $R^8$ - die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^3$, $R^4$, $R^5$, $R^6$ gleich oder verschieden sind und

- Wasserstoff bedeuten, oder
- Cycloalkyl bedeutet, oder
- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,

worin

$R^7$, $R^8$ die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können, oder

- Heteroaryl bedeuten, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$ und $R^8$ die oben angegebene Bedeutung haben,

oder

- Aryl bedeuten, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben, oder

$R^3$ und $R^4$ und/oder $R^5$ und $R^6$ jeweils gemeinsam einen Ring bilden, der gegebenenfalls durch ein Sauerstoffatom, ein Schwefelatom, oder eine Gruppe der Formel $-NR^9$ unterbrochen sein kann,

worin

$R^9$ - für Wasserstoff, Alkyl, Aryl, Carbamoyl oder Alkoxycarbonyl steht,

X - eine Gruppe der Formel $-CH_2-CH_2-$ oder $-CH=CH-$ bedeutet,

A - eine Gruppe der Formel

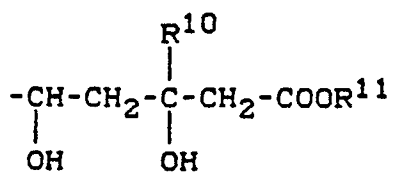

oder

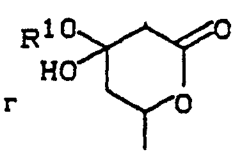

bedeutet,

worin

$R^{10}$ - für Wasserstoff oder Alkyl steht,

und

$R^{11}$ - für Wasserstoff steht, oder

- für Alkyl, Aralkyl oder Aryl steht, oder
- für ein Kation steht,

Y - eine Bindung bedeutet, oder

- eine Gruppe der Formel $-NR^{12}$ bedeutet

worin

$R^{12}$ - für Wasserstoff oder Alkyl steht,

Z - eine Gruppe der Formel $-CH=CH-$ oder $-CH_2-$ bedeutet, oder

- ein Schwefelatom, Sauerstoffatom, oder
- eine Gruppe der Formel $-NR^{12}$ bedeutet,

worin

$R^{12}$ die oben angegebene Bedeutung hat,

und

n und m gleich oder verschieden sind und

- eine Zahl 0, 1, 2, 3 oder 4 bedeuten,

gefunden.

Überraschenderweise zeigen die erfindungsgemäßen substituierten annellierten Pyrrole eine überlegene inhibitorische Wirkung auf die HMG-CoA Reduktase (3-Hydroxy-3-methyl-glutaryl Coenzym A Reduktase).

Cycloalkyl steht im allgemeinen für einen cyclischen Kohlenwasserstoffrest mit 3 bis 8 Kohlenstoffatomen. Bevorzugt ist der Cyclopropyl-, Cyclopentan- und der Cyclohexanring. Beispielsweise seien Cyclopropyl, Cyclopentyl, Cyclohexyl, Cycloheptyl und Cyclooctyl genannt.

Alkyl steht im allgemeinen für einen geradkettigen oder verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis etwa 6 Kohlenstoffatomen. Besonders bevorzugt ist ein Alkylthiorest mit 1 bis 4 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Alkylsulfonyl steht im allgemeinen für einen geradkettigen oder verzweigten Kohlenwasserstoffrest mit 1 bis 12 Kohlenstoffatomen, der über eine $SO_2$-Gruppe gebunden ist. Bevorzugt ist Niederalkylsulfonyl mit 1 bis etwa 6 Kohlenstoffatomen. Beispielsweise seien genannt: Methylsulfonyl, Ethylsulfonyl, Propylsulfonyl, Isopropylsulfonyl, Butylsulfonyl, Isobutylsulfonyl, Pentylsulfonyl, Isopentylsulfonyl, Hexylsulfonyl, Isohexylsulfonyl.

Sulfamoyl (Aminosulfonyl) steht für die Gruppe $-SO_2-NH_2$.

Aryl steht im allgemeinen für einen aromatischen Rest mit 6 bis etwas 12 Kohlenstoffatomen. Bevorzugte Arylreste sind Phenyl, Naphthyl und Biphenyl.

Aryloxy steht im allgemeinen für einen aromatischen Rest mit 6 bis etwa 12 Kohlenstcffatomen, der über ein Sauerstoffatom gebunden ist. Bevorzugte Aryloxyreste sind Phenoxy oder Naphthyloxy.

Arylthio steht im allgemeinen für einen aromatischen Rest mit 6 bis etwa 12 Kohlenstoffatomen, der über ein Schwefelatom gebunden ist. Bevorzugte Arylthioreste sind Phenylthio oder Naphthylthio.

Arylsulfonyl steht im allgemeinen für einen aromatischen Rest mit 6 bis etwa 12 Kohlenstoffatomen, der über eine $SO_2$-Gruppe gebunden ist. Beispielsweise seien genannt: Phenylsulfonyl, Naphthylsulfonyl und Biphenylsulfonyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Aralkylthio steht im allgemeinen für einen Aralkylrest mit 7 bis etwa 14 Kohlenstoffatomen wobei die Alkylkette über ein Schwefelatom gebunden ist. Bevorzugt werden aralkylthioreste mit 1 bis 6 Kohlenstoffatomen im aliphatischen Teil und 6 bis 12 Kohlenstoffatomen im aromatischen Teil. Beispielsweise seien folgende Aralkylthioreste genannt: Benzylthio, Naphthylmethylthio, Phenethylthio und Phenylpropylthio.

Aralkylsulfonyl steht im allgemeinen für einen Aralkylrest mit 7 bis etwa 14 Kohlenstoffatomen, wobei die Alkylreste über eine $SO_2$-Kette gebunden ist. Bevorzugt werden Aralkylsulfonylreste mit 1 bis 6 Kohlenstoffatomen im aliphatischen Teil und 6 bis 12 Kohlenstoffatomen im aromatischen Teil. Beispielsweise seien folgende Aralkylsulfonylreste genannt: Benzylsulfonyl, Naphthylmethylsulfonyl, Phenethlysulfonyl und Phenylpropylsulfonyl.

Alkoxycarbonyl kann beispielsweise durch die Formel

$$-\underset{\underset{O}{\|}}{C}-OAlkyl$$

dargestellt werden. Alkyl steht hierbei für einen geradkettigen oder verzweigten Kohlenwasserstoffrest mit 1 bis 12 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil.

Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Acyl steht im allgemeinen für Phenyl oder geradkettiges oder verzweigtes Niederalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Halogen steht im allgemeinen für Fluor, Chlor, Brom oder Iod, bevorzugt für Fluor, Chlor oder Brom. Besonders bevorzugt steht Halogen für Fluor oder Chlor.

Heteroaryl steht im allgemeinen für einen 5- bis 6-gliedrigen aromatischen Ring, der als Heteroatome Sauerstoff, Schwefel und/oder Stickstoff enthalten kann und an den weitere aromatische Ringe ankondensiert sein können. Bevorzugt sind 5- und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt: Thienyl, Furyl, Pyrrolyl, Pyrazolyl, Pyridyl, Pyrimidyl, Pyrazinyl, Pyridazinyl, Chinolyl, Isochinolyl, Chinazolyl, Chinoxalyl, Phthalazinyl, Cinnolyl, Thiazolyl, Benzothiazolyl, Isothiazolyl, Oxazolyl, Benzoxazolyl, Isoxazolyl, Imidazolyl, Benzimidazolyl, Indolyl, und Isoindolyl.

Im Rahmen der vorliegenden Erfindung entsprechend die substituierten annellierten Pyrrole (Ia) der allgemeinen Formel

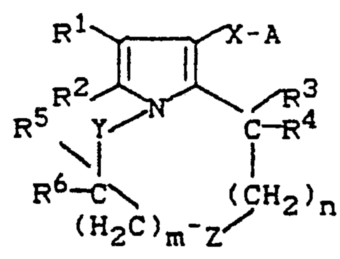

(Ia)

in welcher

$R^1$, $R^2$, $R^3$, $R^4$, $R^5$, $R^6$, X, Y, Z, A, n und m die oben angegebene Bedeutung haben.

Im Rahmen der vorliegenden Erfindung entsprechend die substituierten annellierten Pyrrole (Ib) der allgemeinen Formel

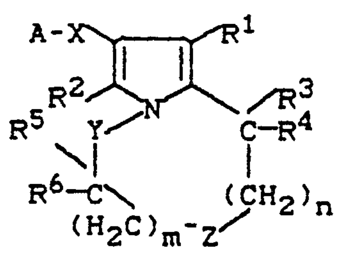

(Ib)

in welcher

$R^1$, $R^2$, $R^3$, $R^4$, $R^5$, $R^6$, X, Y, Z, A, n und m die oben angegebene Bedeutung haben.

Im Rahmen der allgemeinen Formel (I) sind Verbindungen mit den allgemeinen Formeln (Ia) und (Ib) bevorzugt.

Bevorzugt sind Verbindungen der allgemeinen Formel (I)

in welcher

$R^1$ - Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder

- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluorme thylthio, Niederalkoxycarbonyl oder durch eine Gruppe der Formel $-NR^7R^8$,

wobei
$R^7$ und $R^8$ gleich oder verschieden sind und für Niederalkyl, Phenyl, Benzyl, Acetyl, Benzoyl, Phenylsulfonyl oder Niederalkylsulfonyl stehen,
$R^2$ - Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^7R^8$,
wobei
$R^7$ und $R^8$ die oben angegebene Bedeutung haben,
- oder Cyclopropyl, Cyclopentyl oder Cyclohexyl bedeutet, oder
- Niederalkyl bedeutet, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^7R^8$,
worin
$R^7$ und $R^8$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,
$R^3$, $R^4$, $R^5$, $R^6$ gleich oder verschieden sein können, und
- Wasserstoff, oder
- Cyclopropyl, Cyclopentyl oder Cyclohexyl bedeuten, oder
- Niederalkyl bedeuten, dass substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^7R^8$,
worin
$R^7$ und $R^8$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, Niederalkyl, Niederalkoxy, Trifluormethyl, oder Trifluormethoxy substituiert sein können, oder
- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimida zolyl bedeuten, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeuten, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy. Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^7R^8$,
wobei
$R^7$ und $R^8$ die oben angegebene Bedeutung haben,
oder
$R^3$ und $R^4$ und/oder $R^5$ und $R^6$ jeweils gemeinsam einen Ring bilden, der gegebenenfalls durch ein Sauerstoffatom, ein Schwefelatom oder durch eine Gruppe der Formel $-NR^9$ unterbrochen sein kann,
worin
$R^9$ - Wasserstoff, Niederalkyl, Phenyl, Benzyl oder Niederalkoxycarbonyl bedeutet,
X - eine Gruppe der Formel $-CH=CH-$ bedeutet
A - eine Gruppe der Formel

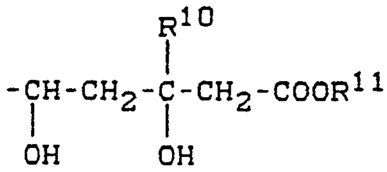 oder 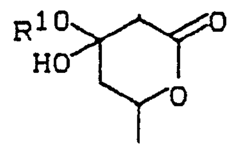

bedeutet,
worin
$R^{10}$ - für Wasserstoff oder Niederalkyl steht,
$R^{11}$ - für Wasserstoff steht, oder
- für Niederalkyl oder Phenyl steht, oder
- für ein physiologisch verträgliches Kation steht, oder
- für Benzyl steht,
Y - eine Bindung bedeutet, oder
- eine Gruppe der Formel $-NR^{12}$ bedeutet,
worin
$R^{12}$-für Wasserstoff oder Niederalkyl steht,
Z - eine Gruppe der Formel -CH = CH- oder $-CH_2-$ bedeutet oder
- ein Schwefelatom, Sauerstoffatom oder
- eine Gruppe der Formel $-NR^{12}$ bedeutet,
worin
$R^{12}$ die oben angegebene Bedeutung hat,
und
n und m gleich oder verschieden sind und
- eine der aufgeführten Zahlen 0, 1, 2, 3 oder 4 bedeuten.

Besonders bevorzugt sind Verbindungen der allgemeinen Formel (I)
in welcher
$R^1$ - Pyridyl, Pyrimidyl, Chinolyl oder Isochinolyl bedeutet, das durch Fluor, Chlor, Methyl, Methoxy oder Trifluormethyl substituiert sein kann, oder
- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
$R^2$ - Pyridyl, Pyrimidyl, Chinolyl oder Isochinolyl bedeutet, das durch Fluor, Chlor, Methyl, Methoxy oder Trifluormethyl substituiert sein kann, oder
- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl, oder
- Cyclopropyl, Cyclopentyl oder Cyclohexyl bedeutet,
oder
- Methyl, Ethyl, Propyl, Isopropyl, Butyl, sec.-Butyl oder tert.Butyl bedeutet, das substituiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
$R^3$, $R^4$, $R^5$ und $R^6$ gleich oder verschieden sind und
- Wasserstoff, Cyclopropyl, Cyclopentyl oder Cyclohexyl bedeuten, oder
- Methyl, Ethyl, Propyl, Isopropyl, Butyl, Isobutyl, tert.Butyl, Pentyl, Isopentyl, Hexyl oder Isohexyl bedeuten, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl,

tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^7R^8$,

wobei

$R^7$ und $R^8$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,

oder durch Pyridyl, Pyrimidyl, Pyrazinyl, Pyridazinyl, Chinolin, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert.Butyl, Methoxy, Ethoxy, Propoxy, Isopropoxy, Butoxy, Isobutoxy, tert.-Butoxy, Trifluormethyl oder Trifluormethoxy substituiert sein können, oder

- Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl bedeuten, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder

- Phenyl bedeuten, das bis zu 3-fach gleich oder ver schieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^7R^8$ substituiert sein kann,

wobei

$R^7$ und $R^8$ die oben angegebene Bedeutung haben, oder

$R^3$ und $R^4$ und/oder $R^5$ und $R^6$ jeweils gemeinsam einen Ring bilden, der gegebenenfalls durch ein Sauerstoffatom, ein Schwefelatom oder durch eine Gruppe der Formel $-NR^9$ unterbrochen sein kann,

worin

$R^9$ - für Wasserstoff, Methyl, Ethyl, Propyl, Isopropyl, tert.Butyl, Phenyl, Benzyl, Methoxycarbonyl, Ethoxycarbonyl, Isopropoxycarbonyl, Propoxycarbonyl, Butoxycarbonyl, Isobutoxycarbonyl oder tert.Butoxycarbonyl steht,

X - eine Gruppe der Formel -CH=CH- bedeutet,

A - eine Gruppe der Formel

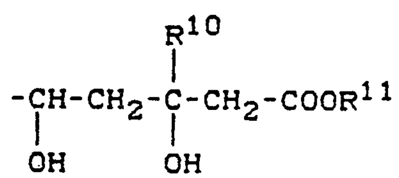

oder

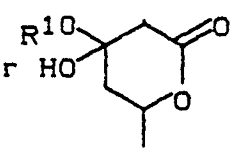

bedeutet,

worin

$R^{10}$ - für Wasserstoff, Methyl, Ethyl, Propyl, Isopropyl, Butyl, Isobutyl oder tert.Butyl steht,

$R^{11}$ - für Wasserstoff, Methyl, Ethyl, Propyl, Isopropyl, Butyl, Isobutyl, tert.Butyl oder Benzyl steht, oder

- für ein Natrium-, Kalium-, Calcium- oder Magnesium- oder Ammoniumion steht,

Y - eine Bindung bedeutet, oder

- eine Gruppe der Formel $-NR^{12}$ bedeutet,

worin

$R^{12}$ - für Wasserstoff, Methyl, Ethyl, Propyl, Isopropyl oder tert.Butyl steht,

Z - eine Gruppe der Formel -CH=CH- oder $-CH_2-$ bedeutet, oder

- ein Schwefelatom, Sauerstoffatom oder eine Gruppe der Formel $-NR^{12}$ bedeutet,

worin

$R^{12}$ die oben angegebene Bedeutung hat,

und

m und n gleich oder verschieden sind und
- eine der aufgeführten Zahlen 0, 1, 2, 3 oder 4 bedeuten.

Ganz besonders bevorzugt sind Verbindungen der allgemeinen Formeln (Ia) und (Ib)
in welcher
$R^1$ - Phenyl bedeutet, das bis zu 2-fach gleich oder verschieden durch Methyl, Ethyl, Propyl, Isopropyl, Butyl, Isobutyl, tert.Butyl, Methoxy, Ethoxy, Propoxy, Isopropoxy, Phenoxy, Benzyloxy, Fluor, Chlor, Brom, Cyano, Trifluormethyl oder Trifluormethoxy substituiert sein kann,
$R^2$ - Phenyl bedeutet, das bis zu 2-fach gleich oder verschieden durch Methyl, Ethyl, Propyl, Isopropyl, Butyl, Isobutyl, tert.Butyl, Methoxy, Ethoxy, Propoxy, Isopropoxy, Phenoxy, Benzyloxy, Fluor, Chlor, Brom, Cyano, Trifluormethyl oder Trifluormethoxy substituiert sein kann, oder
- Cyclopropyl, Methyl, Ethyl, Propyl, Isopropyl, Butyl, Isobutyl oder tert.Butyl bedeutet, das durch Fluor, Chlor, Methoxy, Phenyl oder Phenoxy substituiert sein kann,
$R^3$, $R^4$, $R^5$, $R^6$ gleich oder verschieden sind und
- Wasserstoff bedeuten, oder
- Methyl, Ethyl, Propyl, Isopropyl bedeuten,
X - eine Gruppe der Formel

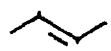 (E-konfiguriert)

bedeutet
und
A - eine Gruppe der Formel

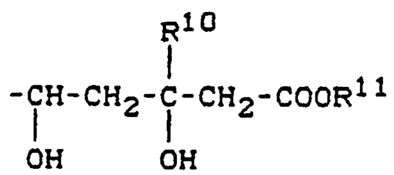 oder 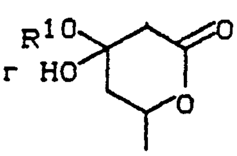

bedeutet,
worin
$R^{10}$ - für Wasserstoff steht und
$R^{11}$ - für Wasserstoff, Methyl oder Ethyl steht, oder
- für ein Natrium- oder Kaliumkation steht,
Y - eine Bindung bedeutet,
Z - eine Gruppe der Formel $-CH_2-$ bedeutet,
n - 0 bedeutet und
m - 1 und 2 bedeutet.

Die erfindungsgemäßen substituierten anellierten Pyrrole der allgemeinen Formel (I) haben mehrere asymmetrische Kohlenstoffatome und können daher in verschiedenen stereochemischen Formel existieren. Sowohl die einzelnen Isomeren als auch deren Mischungen sind Gegenstand der Erfindung.

Je nach der Bedeutung der Gruppen X bzw. der Reste A ergeben sich unterschiedliche Stereoisomere, die im folgenden näher erläutert werden sollen:

In den folgenden Erläuterungen wird der Rest

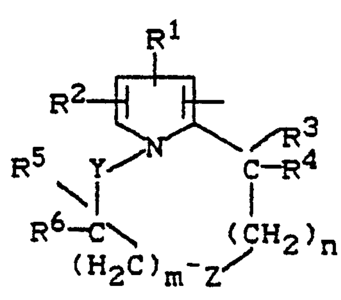

durch das Symbol

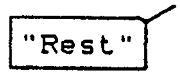

vereinfacht dargestellt.

a) Steht die Gruppe -X- für eine Gruppe der Formel -CH=CH- , so können die erfindungsgemäßen Verbindungen in zwei stereoisomeren Formen existieren, die an der Doppelbindung E-konfiguriert (II) oder Z-konfiguriert (III) sein können:

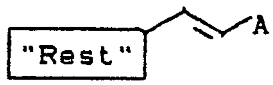

(II) E-Form

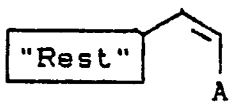

(III) Z-Form

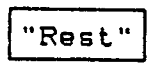

und A haben die oben angegebene Bedeutung.

Bevorzugt sind diejenigen Verbindungen der allgemeinen Formel (I) die E-konfiguriert sind (II).

b) Steht der Rest -A- für eine Gruppe der Formel

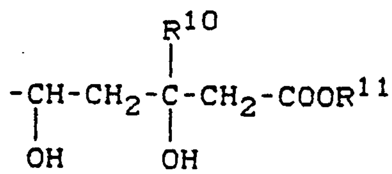

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

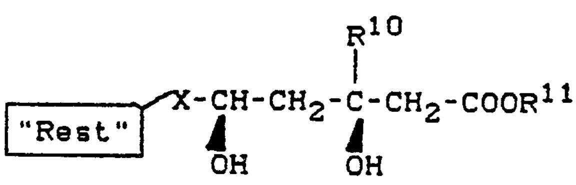

erythro-Form (IV)

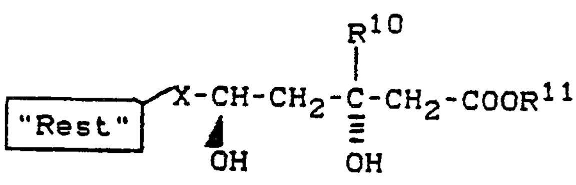

threo-Form (V)

Sowohl von den Verbindungen in erythro- als auch in threo-Konfiguration existieren wiederum jeweils zwei Enantiomere, nämlich 3R,5S-Isomeres bzw. 3S,5R-Isomeres (erythro-Form) sowie 3R,5R-Isomeres und 3S,5S-Isomeres (threo-Form).

Bevorzugt sind hierbei die erythro-konfigurierten Isomeren, besonders bevorzugt das 3R,5S-Isomere sowie das 3R,5S-3S,5R-Racemat.

c) Steht der Rest -A für eine Gruppe der Formel

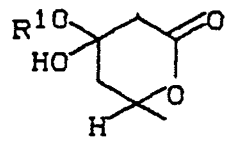

so besitzen die substituierten anellierten Pyrrole mindestens zwei asymmetrische Kohlenstoffatome, nämlich das Kohlenstoffatom an dem die Hydroxygruppe gebunden ist, und das Kohlenstoffatom an welchem der Rest der Formel

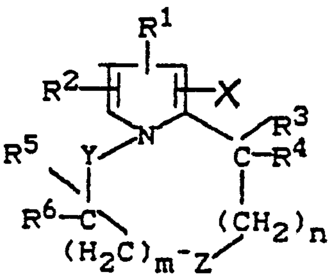

[dargestellt durch das Symbol "Rest"]

gebunden ist. Je nach der Stellung der Hydroxygruppe zur freien Valenz am Lactonring können die substituierten anellierten Pyrrole als cis-Lactone (VI) oder als trans-Lactone (VII) vorliegen.

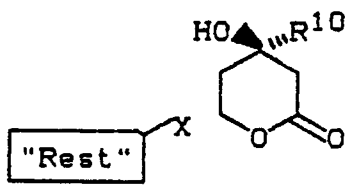

cis-Lacton (VI)

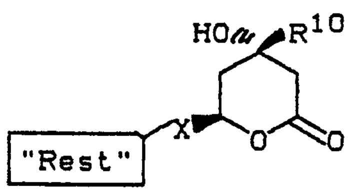

trans-Lacton (VII)

Sowohl vom cis-Lacton aus als auch vom trans-Lacton existieren wiederum jeweils zwei Isomere nämlich das 4R,6R-Isomere bzw. das 4S,6S-Isomere (cis-Lacton), sowie das 4R,6S-Isomere bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten anellierten Pyrrole genannt:

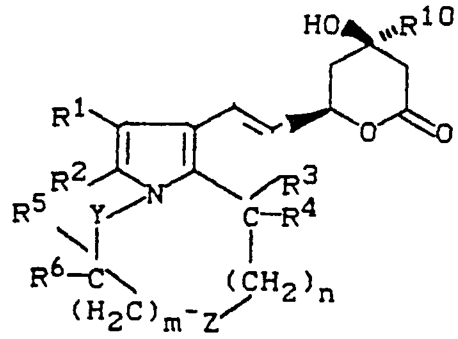

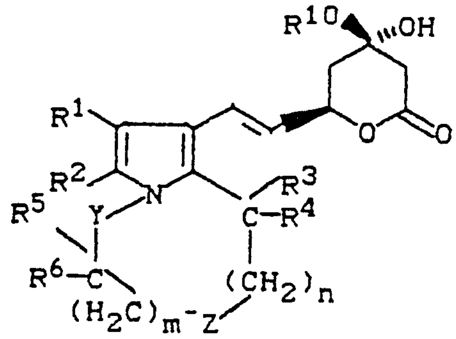

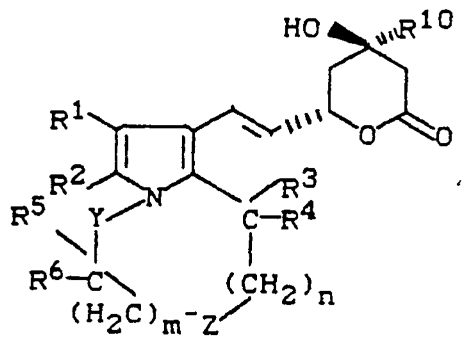

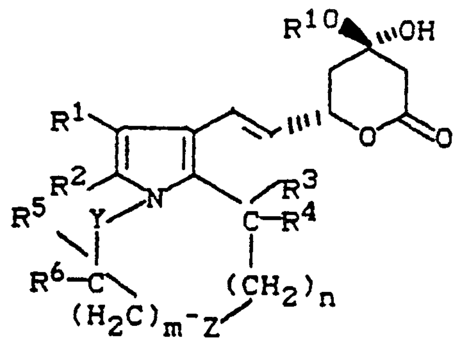

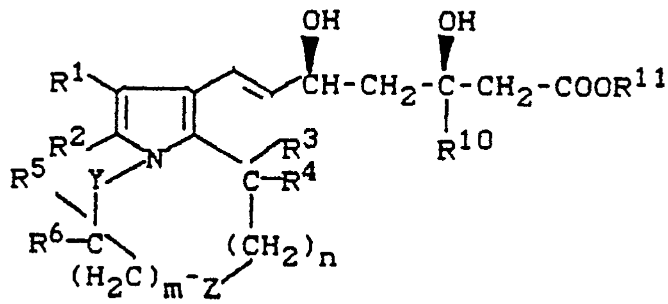

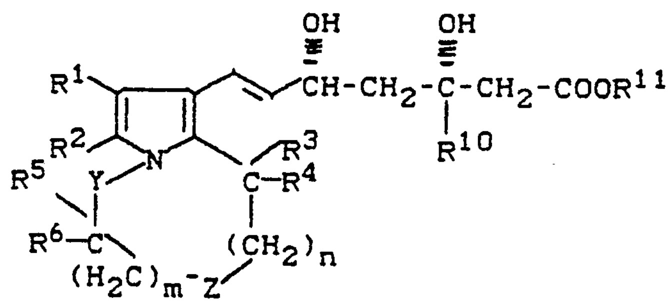

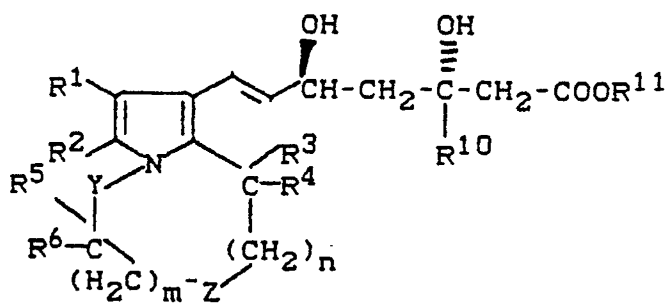

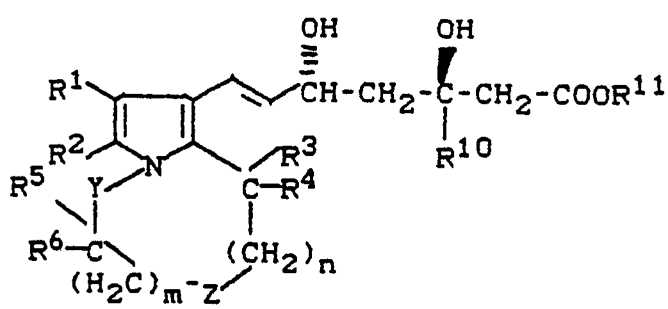

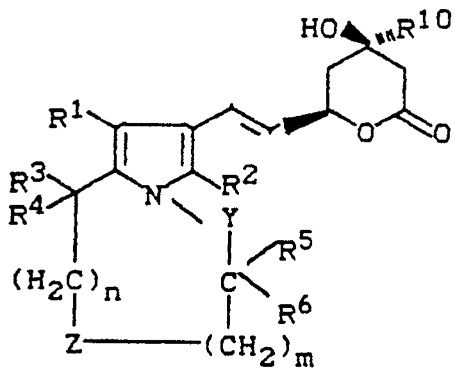

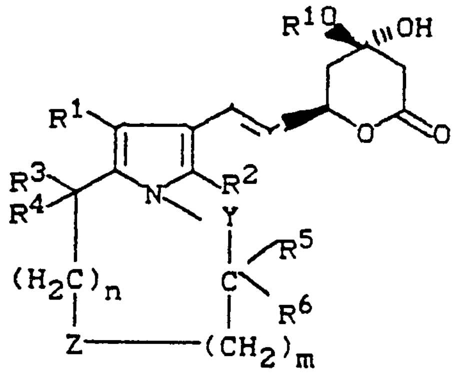

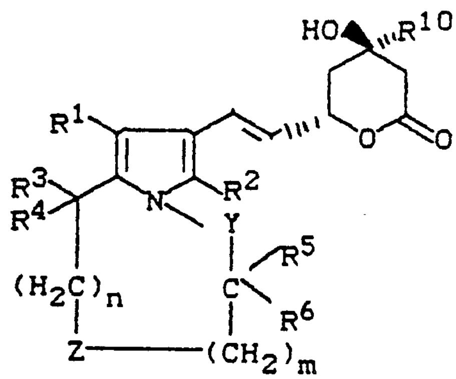

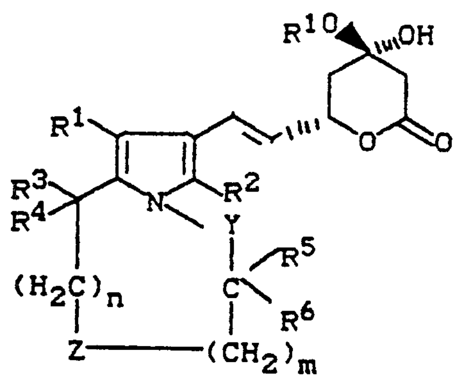

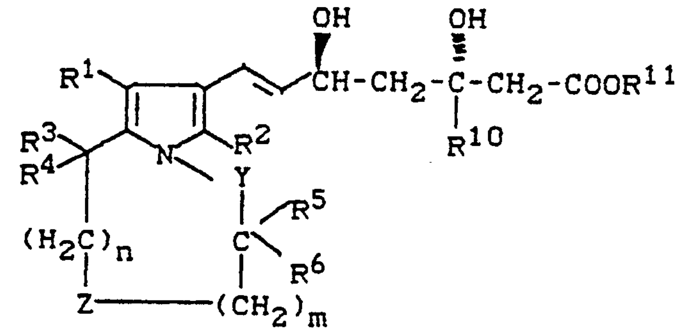

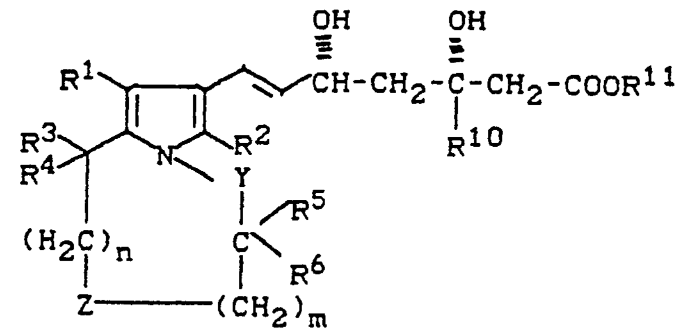

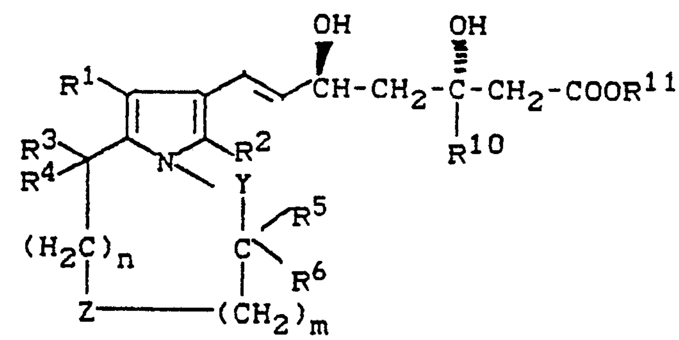

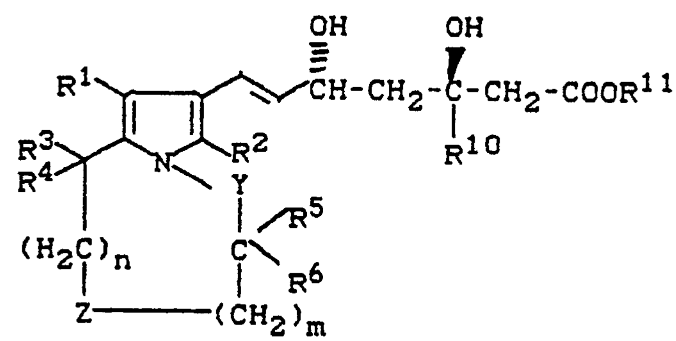

Darüberhinaus ergeben sich weitere Möglichkeiten der Isomerenbildung, da die erfindungsgemäßen substituierten annellierten Pyrrole weitere Chiralitätszentren enthalten können. Es sind ebenso alle Stereoisomeren Gegenstand der Erfindung, die durch die weiteren Chiralitätszentren, insbesondere im Zusammenhang mit der Gruppe -X-A entstehen.

Außerdem wurde ein Verfahren zur Herstellung der substituierten anellierten Pyrrole der allgemeinen Formel (I),

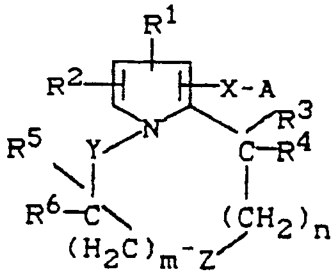

(I)

in welcher
$R^1$, $R^2$, $R^3$, $R^4$, $R^5$, $R^6$, A, X, Y, Z, m und n die oben angegebene Bedeutung haben, gefunden, das dadurch gekennzeichnet ist, daß man
Ketone der allgemeinen Formel (VIII)

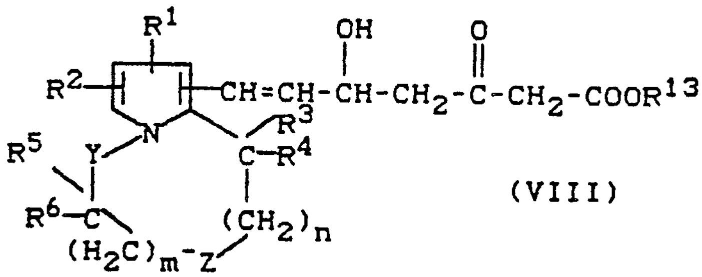

(VIII)

in welcher
$R^1$, $R^2$, $R^3$, $R^4$, $R^5$, $R^6$, Y, Z, m und n die oben angegebene Bedeutung haben
und
$R^{13}$ - für Alkyl steht,
reduziert,
und im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethanverbindung (X = $-CH_2-CH_2-$) die Ethenverbindung (X = -CH=CH-) nach üblichen Methoden hydriert,
und gegebenenfalls Isomere trennt.

Das erfindungsgemäße Verfahren kann durch das folgende Formelschema erläutert werden:

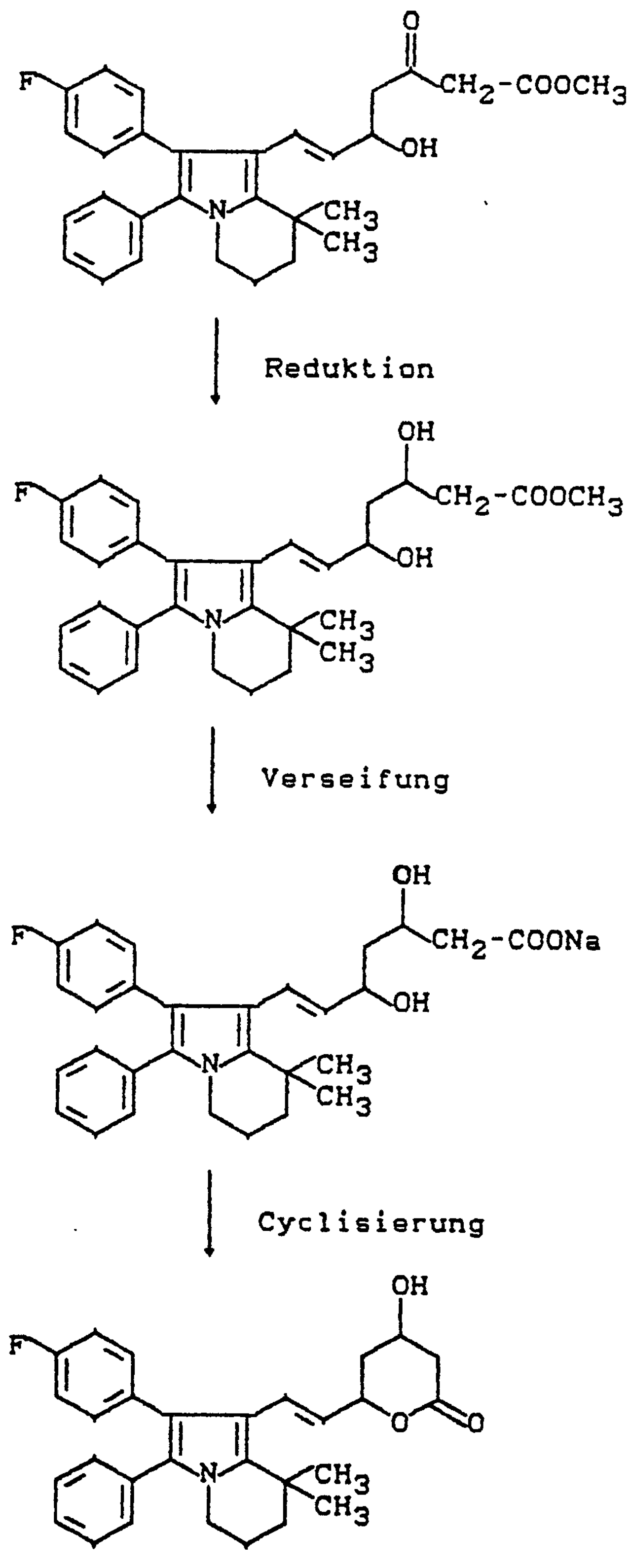


Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-borate, Natrium-trialkyl-hydrido-boranaten, Natrium-cyano-trihydrido-borat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischem Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispiels weise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80°C bis +30°C, bevorzugt von -78°C bis 0°C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (VIII) unter solchen Bedingungen durchgeführt werden, unter denen sowohl die Carbonylgruppe als auch die Doppelbindung reduziert wird.

Darüberhinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ic)

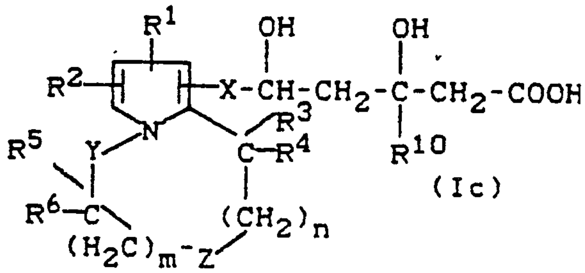

(Ic)

in welcher

$R^1$, $R^2$, $R^3$, $R^4$, $R^5$, $R^6$, $R^{10}$, X, Y, Z, n und m die oben angegebene Bedeutung haben.

Die Carbonsäureester im Rahmen der allgemeinen Formel (I) entsprechen der Formel (Id)

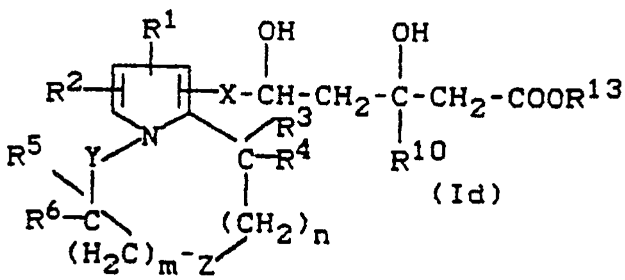

(Id)

in welcher

$R^1$, $R^2$, $R^3$, $R^4$, $R^5$, $R^6$, $R^{10}$, X, Y, Z, m und n die oben angegebene Bedeutung haben und

$R^{13}$ - für Alkyl steht.

Die Salze der erfindungsgemäßen Verbindungen im Rahmen der allgemeinen Formel (I) entsprechen der Formel (Ie)

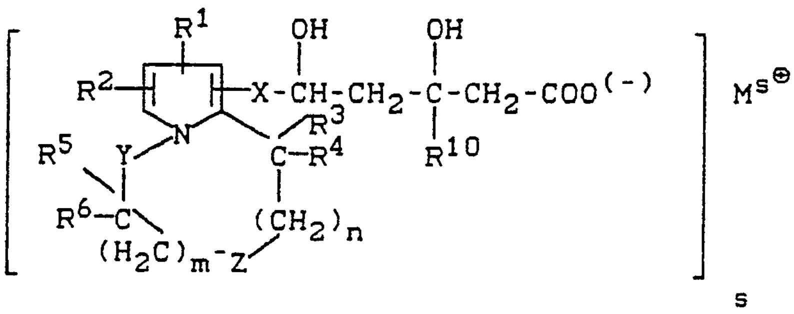

(Ie)

in welcher
$R^1$, $R^2$, $R^3$, $R^4$, $R^5$, $R^6$, $R^{10}$, X, Y, Z, m und n die oben angegebene Bedeutung haben und
$M^{s\oplus}$ für ein Kation steht.

Die Lactone im Rahmen der allgemeinen Formel (I) entsprechen der Formel (If)

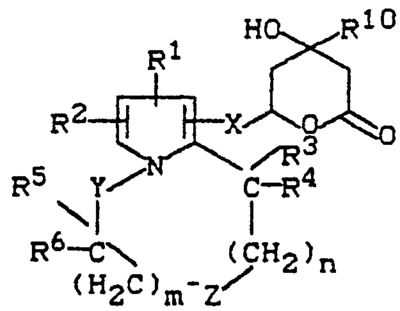

(If)

in welcher
$R^1$, $R^2$, $R^3$, $R^4$, $R^5$, $R^6$, $R^{10}$, X, Y, Z, m und n die oben angegebene Bedeutung haben.

Zur Herstellung der erfindungsgemäßen Carbonsäuren der allgemeinen Formel (Ic) werden im allgemeinen die Carbonsäureester der allgemeinen Formel (Id) oder die Lactone der allgemeinen Formel (If) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ie) entstehen, die anschließend in einem zweiten Schritt durch Behandeln mit Säure in die freien Säuren der allgemeinen Formel (Ic) überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Menge der Reaktanden.

Bei der Durchführung der Reaktion entstehen im ersten Schritt die Salze der erfindungsgemäßen Verbindungen (Ie) als Zwischenprodukte, die isoliert werden können. Die erfindungsgemäßen Säuren (Ic) erhält man durch Behandeln der Salze (Ie) mit üblichen anorganischen Säuren. Hierzu gehören bevorzugt Mineralsäuren wie beispielsweise Chlorwasserstoffsäure, Bromwasserstoffsäure, Schwefelsäure oder

Phosphorsäure. Es hat sich bei der Herstellung der Carbonsäuren (Ic) hierbei als vorteilhaft erwiesen, die basische Reaktionsmischung der Verseifung in einem zweiten Schritt ohne Isolierung der Salze anzusäueren. Die Säuren können dann in üblicher Weise isoliert werden.

Zur Herstellung der erfindungsgemäßen Lactone der Formel (If) werden im allgemeinen die erfindungsgemäßen Carbonsäuren (Ic) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40 °C bis +200 °C, bevorzugt von -25 °C bis +50 °C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüber hinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N′-Dicyclohexylcarbodiimid-Paratoluolsulfonat, N-Cyclohexyl-N′-[2-(N″-methylmorpholinium)ethyl]carbodiimid oder N-(3-Dimethylaminopropyl)-N′-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylen chlorid, Chloroform oder Tetrachlorkohlenstoff eingesetzt.

Die Reaktion wird im allgemeinen in einem Temperaturbereich von 0 °C bis +80 °C, bevorzugt von +10 °C bis +50 °C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Ester. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ig)

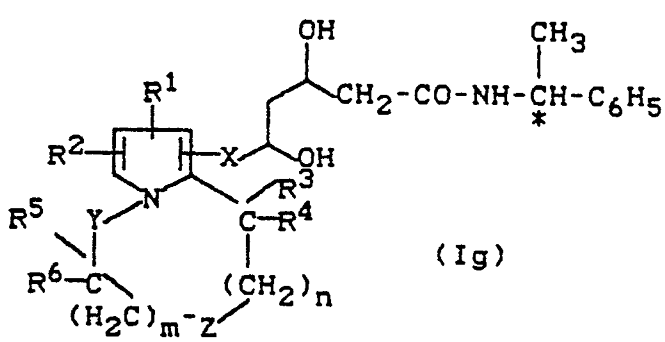

(Ig)

in welcher

$R^1$, $R^2$, $R^3$, $R^4$, $R^5$, $R^6$, X, Y, Z, m und n die oben angegebene Bedeutung haben,

überführt, die anschließend wie üblich durch Chromatographie oder Kristallisation in die einzelnen Diastereomerens getrennt werden können. Anschließend Hydrolyse der reinen diastereomeren Amide nach üblichen Methoden, beispielsweise durch Behandeln der diastereomeren Amide mit anorganischen Basen wie Natriumhydroxid oder Kaliumhydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ic), die wie oben beschrieben durch Cyclisierung in die enantiomerenreinen Lactone überführt

werden können.

Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

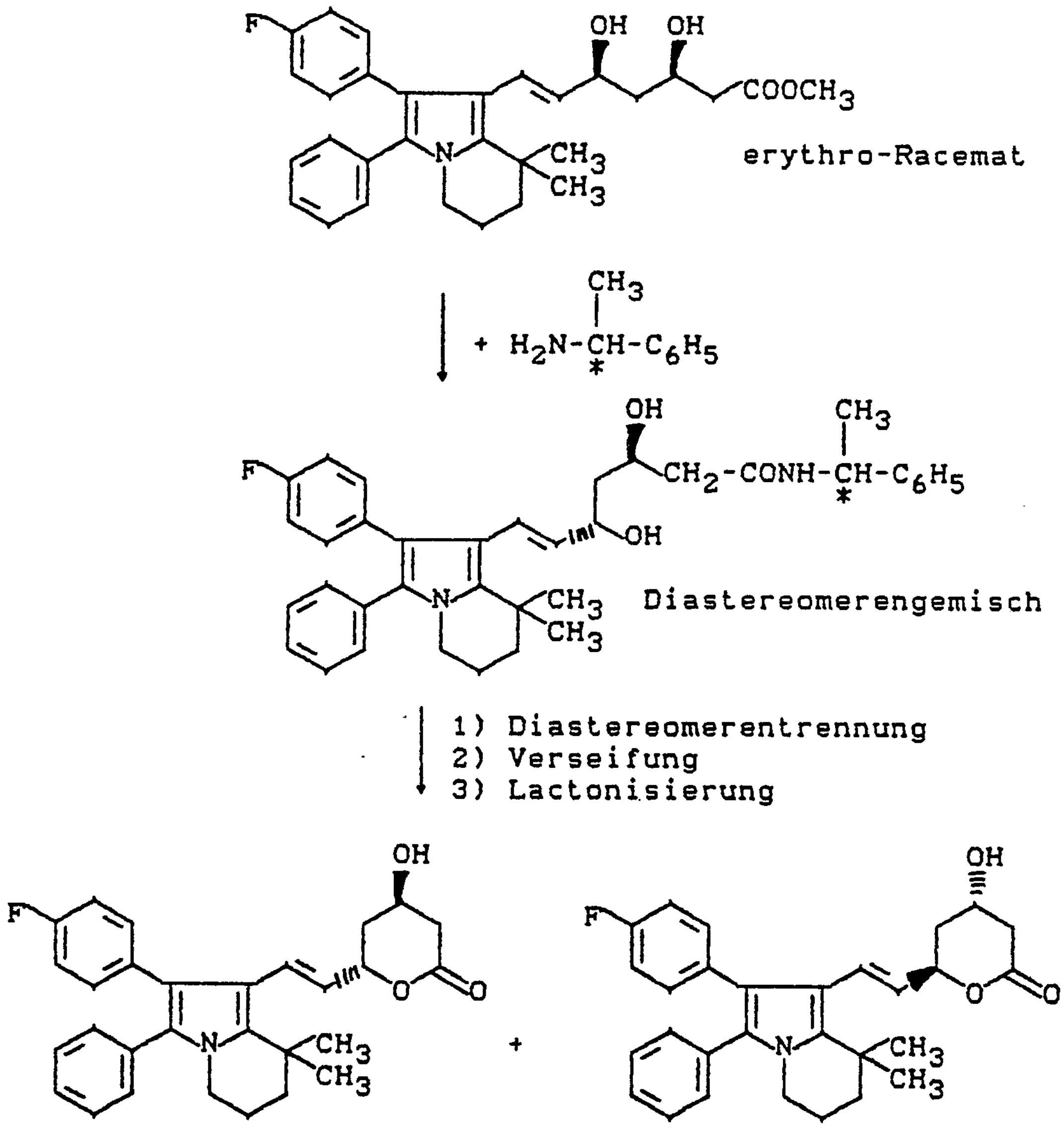


Die als Ausgangsstoffe eingesetzten Ketone (VIII) sind neu.

Es wurde ein Verfahren zur Herstellung der erfindungsgemäßen Ketone der allgemeinen Formel (VIII)

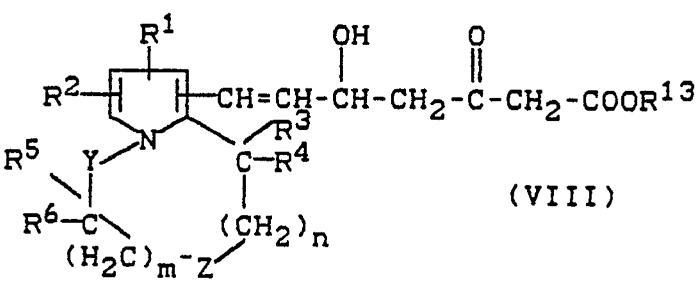

(VIII)

in welcher

$R^1$, $R^2$, $R^3$, $R^4$, $R^5$, $R^6$, $R^{13}$, Y, Z, m und n die oben angegebene Bedeutung haben,
gefunden, das dadurch gekennzeichnet ist, daß man
Aldehyde der allgemeinen Formel (IX)

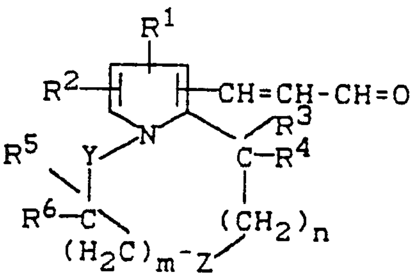

(IX)

in welcher
$R^1$, $R^2$, $R^3$, $R^4$, $R^5$, $R^6$, Y, Z, m und n die oben angegebene Bedeutung haben,
in inerten Lösemitteln mit Acetessigestern der allgemeinen Formel (X)

$H_3C-\overset{\overset{O}{\|}}{C}-CH_2-COOR^{13}$ (X)

in welcher
$R^{13}$ die oben angegebene Bedeutung hat,
in Anwesenheit von Basen umsetzt.

Das erfindungsgemäße Verfahren kann beispielsweise durch folgendes Formelschema erläutert werden:

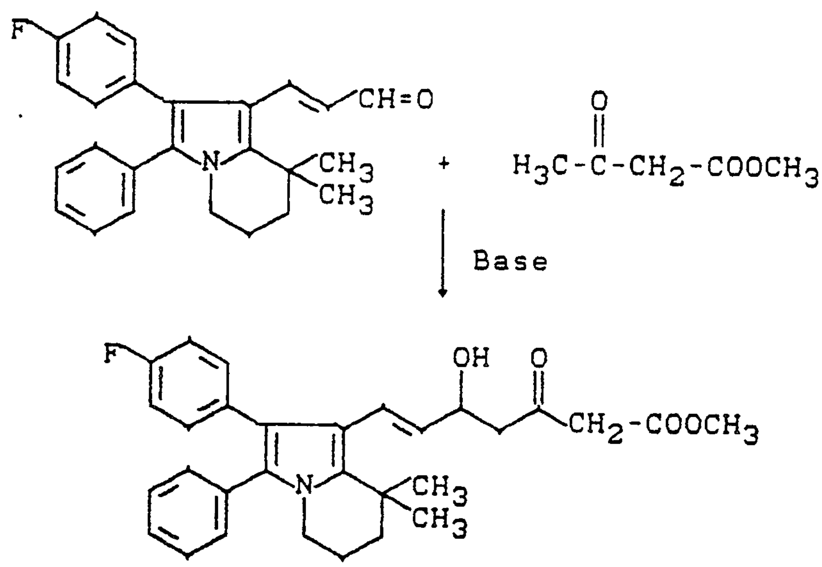


Als Basen kommen hierbei die üblichen stark basischen Verbindungen in Frage. Hierzu gehören bevorzugt lithiumorganische Verbindungen wie beispielsweise n-Butyllithium, sec.Butyllithium, tert.Butyllithium oder Phenylli thium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt werden n-Butyllithium oder Natriumhydrid oder deren Gemisch eingesetzt.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80 °C bis +50 °C, bevorzugt von -20 °C bis +30 °C durchgeführt.

Das Verfahren wird im allgemeinen bei Normaldruck durchgeführt, es ist aber auch möglich das

Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:

Acetessigsäuremethylester,
Acetessigsäureethylester,
Acetessigsäurepropylester,
Acetessigsäueisopropylester.

Die als Ausgangsstoffe eingesetzten Aldehyde der allgemeinen Formel (IX) sind neu.

Es wurde außerdem ein Verfahren zur Herstellung der Aldehyde der allgemeinen Formel (IX)

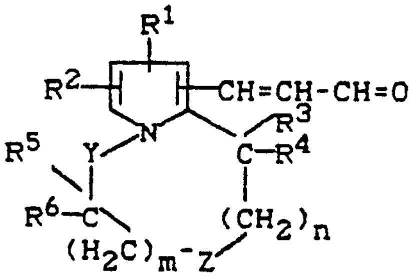

(IX)

in welcher
$R^1$, $R^2$, $R^3$, $R^4$, $R^5$, $R^6$, Y, Z, m und n die oben angegebene Bedeutung haben,
gefunden, das dadurch gekennzeichnet ist, daß man
substituierte Pyrrole der allgemeinen Formel (XI)

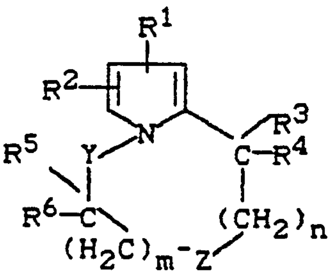

(XI)

in welcher
$R^1$, $R^2$, $R^3$, $R^4$, $R^5$, $R^6$, Y, Z, m und n die oben angegebene Bedeutung haben,
in inerten Lösemitteln in Anwesenheit von Hilfsstoffen mit N,N-Dialkylaminoacrolein der Formel (XII)

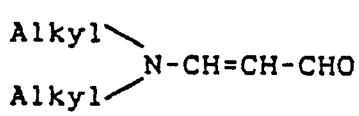

(XII)

wobei
Alkyl- für einen geradkettigen oder verzweigten Kohlenwasserstoffrest mit 1 bis 6 Kohlenstoffatomen steht,
umsetzt.

Das erfindungsgemäße Verfahren kann beispielsweise durch folgendes Formelschema verdeutlicht werden:

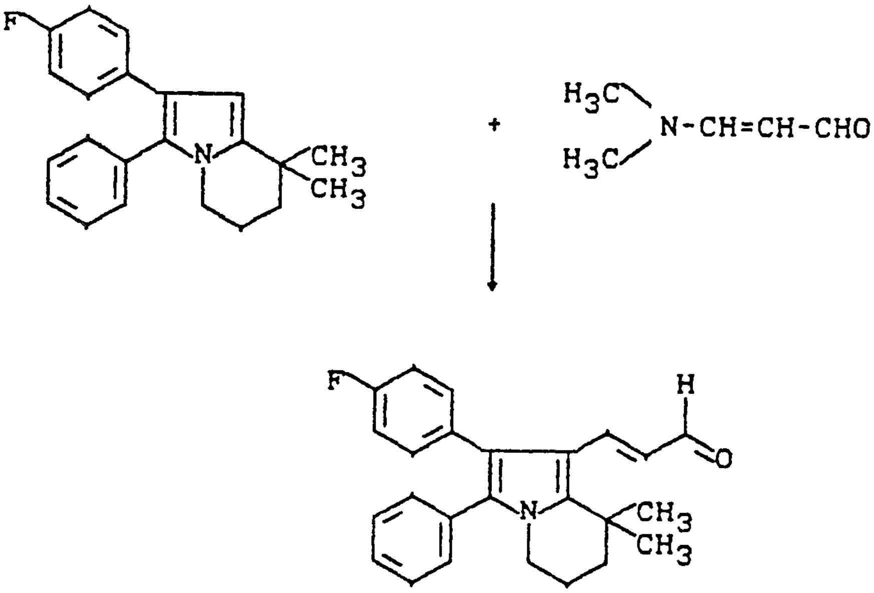

Als Lösemittel eignen sich hierbei die üblichen organischen Lösemittel, die unter den Reaktionsbedingungen stabil sind. Hierzu gehören bevorzugt Kohlenwasserstoffe wie Benzol, Toluol, Xylol, Hexan, Erdölfraktionen, Chlorbenzol oder Dichlorbenzol, oder Ether wie Diethylether, Dioxan oder Tetrahydrofuran, oder Chlorkohlenwasserstoffe wie Methylenchlorid, Chloroform oder Tetrachlorkohlenstoff oder Acetonitril. Ebenso ist es möglich, Gemische der genannten Lösemittel einzusetzen. Besonders bevorzugt wird wasserfreies Acetonitril oder Chloroform verwendet.

Als Hilfsstoffe werden im allgemeinen Säurechloride verwendet. Bevorzugt wird Phosphoroxychlorid oder Phosgen, besonders bevorzugt Phosphoroxychlorid eingesetzt.

Die Reaktion wird in einem Temperaturbereich von -20°C bis +150°C, bevorzugt von 0°C bis +100°C durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung des Verfahrens wird das Dimethylaminoacrolein im allgemeinen in einer Menge von 1 bis 6, bevorzugt von 1 bis 2 mol, bezogen auf 1 mol des Pyrrols eingesetzt.

Die als Ausgangsstoffe eingesetzten substituierten annellierten Pyrrole der allgemeinen Formel (XI) sind an sich bekannt (J. Med. Chem. 1987, 30, 820-823).

Es wurde ein Verfahren zur Herstellung der substituierten anellierten Pyrrole der allgemeinen Formel (XI)

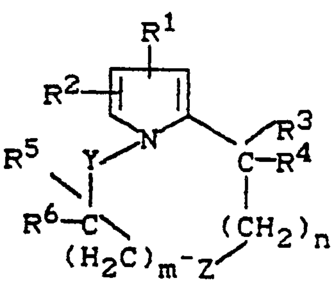 (XI)

in welcher

$R^1$, $R^2$, $R^3$, $R^4$, $R^5$, $R^6$, Y, Z, m und n die oben angegebene Bedeutung haben, gefunden das dadurch gekennzeichnet ist, daß man

Diketone der allgemeinen Formel (XIII), (XIV), (XV) und (XVI)

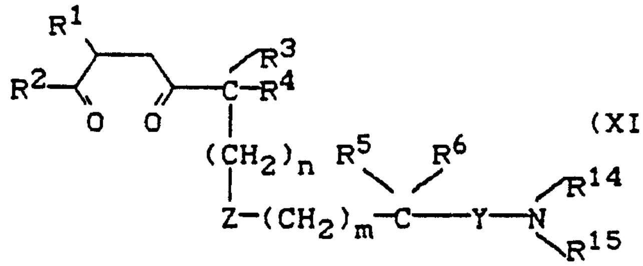

(XIII)

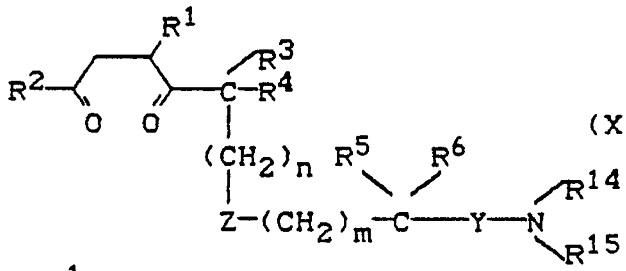

(XIV)

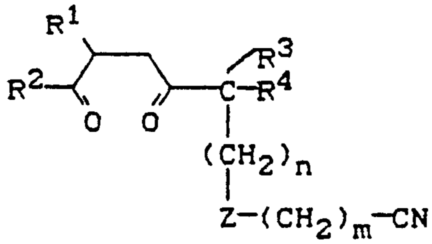

(XV)

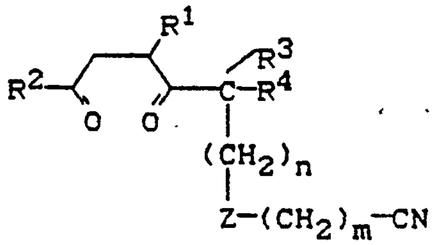

(XVI)

in welchen
$R^1$, $R^2$, $R^3$, $R^4$, $R^5$, $R^6$, Y, Z, m und n die oben angegebene Bedeutung haben,
und
$R^{14}$ und $R^{15}$ gleich oder verschieden sind und
- die für primäre Amine üblichen Schutzgruppen wie Acyl, Phthalimido, Benzyl oder tert.Butoxycarbonyl bedeuten,
in organischen Lösemitteln in Anwesenheit von Hilfsstoffen wie Wasserstoff, Hydrierkatalysatoren, Säuren und Wasser, zu Aminodiketonen der allgemeinen Formel (XVII) und (XVIII)

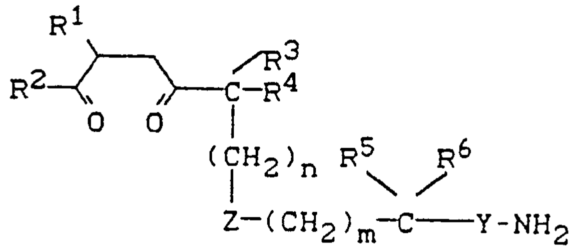

(XVII)

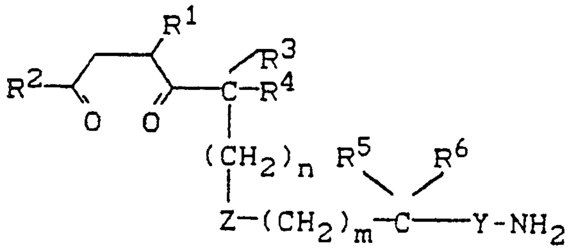

(XVIII)

in welchen
$R^1$, $R^2$, $R^3$, $R^4$, $R^5$, $R^6$, Y, Z, m und n die oben angegebene Bedeutung haben,
umsetzt, die unter den Reaktionsbedingungen zu den substituierten anellierten Pyrrolen (XI) cyclisieren.

Als Lösemittel eignen sich hierei die üblichen organischen Lösemittel, die unter den Reaktionsbedingungen stabil sind. Hierzu gehören bevorzugt Kohlenwasserstoffe wie Benzol, Toluol, Xylol, Hexan, Cyclohexan, Erdölfraktionen, oder Ether wie Diethylether, Dioxan, Tetrahydrofuran oder Alkohole wie Methanol, Ethanol oder Propanol. Ebenso ist es möglich, Gemische der genannten Lösemittel einzusetzen. Besonders bevorzugt wird Methanol oder Ethanol verwendet.

Als Hilfstoffe werden im allgemeinen Wasserstoff und Hydrierkatalysatoren eingesetzt. Bevorzugt wird Raney-Kobalt und Wasserstoff eingesetzt.

Die Reaktion wird in einem Temperaturbereich von -20 °C bis +250 °C, bevorzugt von 0 °C bis +100 °C durchgeführt.

Das Verfahren wird im allgemeinen unter Druck durchgeführt. Bevorzugt wird dabei der Bereich von 1 bis 300 bar, besonders bevorzugt der Bereich von 20 - 80 bar.

Die als Ausgangsstoffe eingesetzten Diketone der allgemeinen Formel (XIII), (XIV), (XV) und (XVI) sind teilweise neu oder können nach bekannten Methoden hergestellt werden [Angew. Chemie, 88, 695 796 (1976)].

Für den Fall, daß $R^3$ verschieden ist von $R^4$ und/oder $R^5$ eine andere Bedeutung hat als $R^6$, können weitere Stereoisomere auftreten.

Enantiomerenreine Formen der allgemeinen Formel (IX) erhält man z.B., indem man Diastereomerengemische der Verbindungen der allgemeinen Formel (IXX)

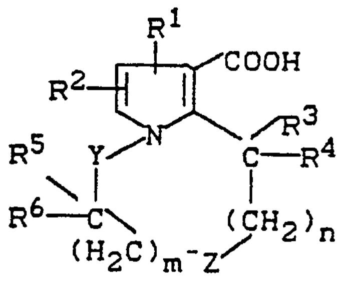

(IXX)

in welcher
$R^1$, $R^2$, $R^3$, $R^4$, $R^5$, $R^6$, Y, Z, m und n die oben angegebene Bedeutung haben,
in stereoisomer einheitliche Bestandteile nach üblichen Methoden, wie sie beispielsweise von E.L. Eliel, Stereochemistry of Carbon Compounds, Mc Graw Hill, 1962 beschrieben werden, trennt.

Die Trennung kann z.B. über Salze oder Amide mit chiralen Basen erfolgen. Durch anschließende Chromatographie oder Kristallisation können die enantiomerenreinen Carbonsäuren der allgemeinen Formel (IXXa), (IXXb), (IXXc) und (IXXd)

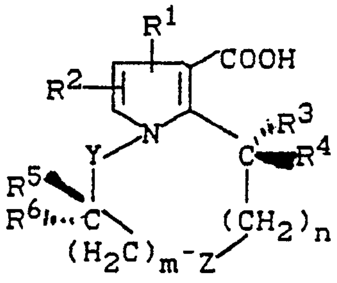 (IXXa)

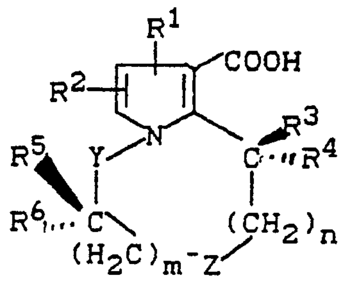 (IXXb)

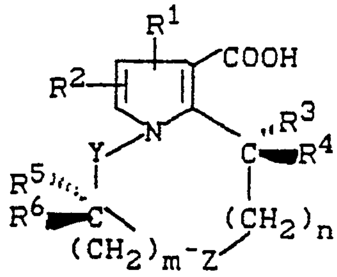 (IXXc)

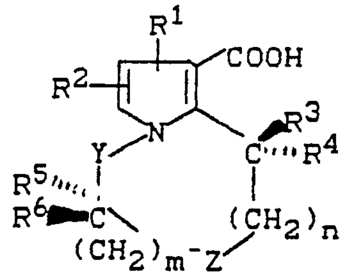 (IXXd)

in welcher
$R^1$, $R^2$, $R^3$, $R^4$, $R^5$, $R^6$, Y, Z, m und n die oben angegebene Bedeutung haben,
hergestellt werden.

Nach üblichen Methoden werden die Verbindungen der allgemeinen Formeln (IXXa), (IXXb), (IXXc) und (IXXd) zunächst zum Alkohol reduziert, oxidiert und anschließend einer Formylolefinierung mit Phosphonsäure-[2-(Cyclohexylamino)vinyl]-diethylester unterzogen.

Die Verbindungen der allgemeinen Formel (IXX) sind teilweise bekannt oder neu und können aus den Verbindungen der allgemeinen Formel (XI) nach üblicher Methode hergestellt werden [C. Ferri, Reaktionen der organischen Synthese, Georg Thieme Verlag, Stuttgart, 1978].

Zur Trennung des Diastereomerengemisches der allgemeinen Formel (IXX) können chirale Basen benutzt werden. Bevorzugt sind Chinin, Cinchonin, Cinchonidin, Brucin, D-(+)- oder L-(-)-α-Methyl-benzylamin.

Für die Reduktion der Verbindungen der allgemeinen Formeln (IXXa), (IXXb), (IXXc) und (IXXd) werden Lithiumaluminiumhydrid
oder Diisobutylaluminiumhydrid eingesetzt. Bevorzugt ist Diisobutylaluminiumhydrid.

Die Oxidation erfolgt nach üblicher Methode [vgl. C. Ferri, Reaktionen der organischen Synthese, Georg Thieme Verlag, Stuttgart, 1978]. Bevorzugt wird Pyridiniumchlorochromat eingesetzt.

Phosphonsäure-[2-(Cyclohexylamino)vinyl]-diethylester ist bekannt [Tetrahedron Lett. 1968, (41), 4359-4362].

Der Herstellungsprozeß für die erfindungsgemäß enantiomerenreinen Formen kann durch die folgenden Reaktionsschemata dargestelt werden:

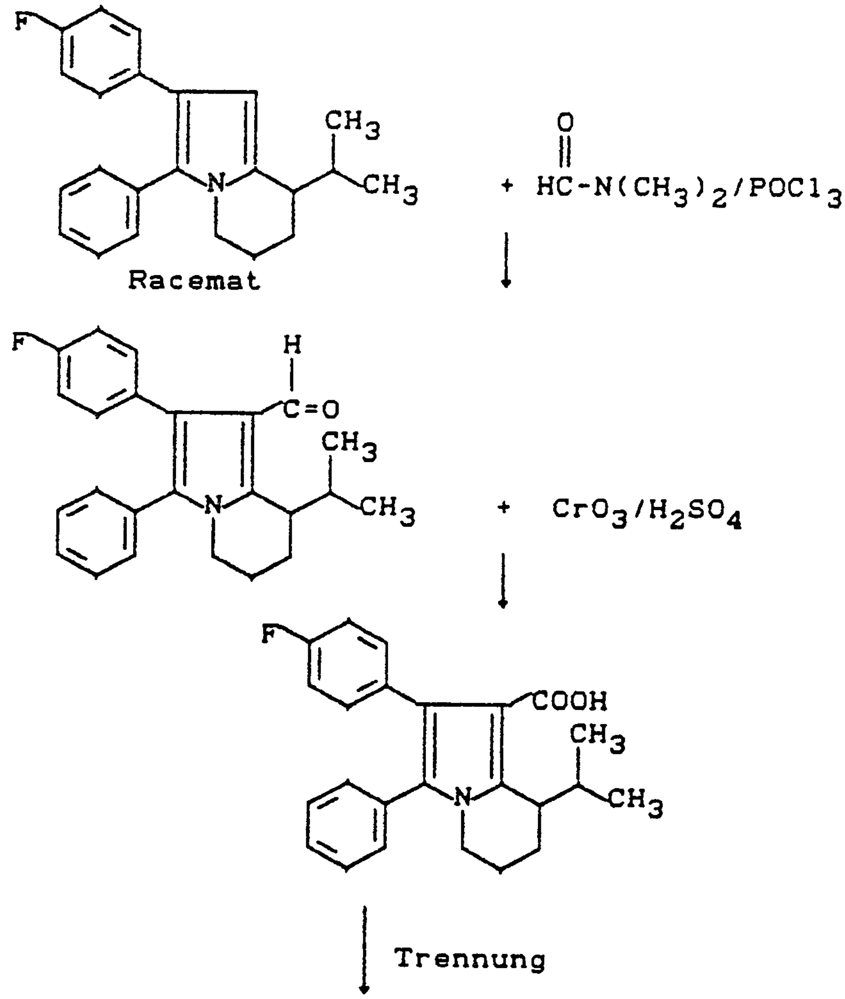
Racemat
+ HC-N(CH3)2/POCl3
+ CrO3/H2SO4
Trennung

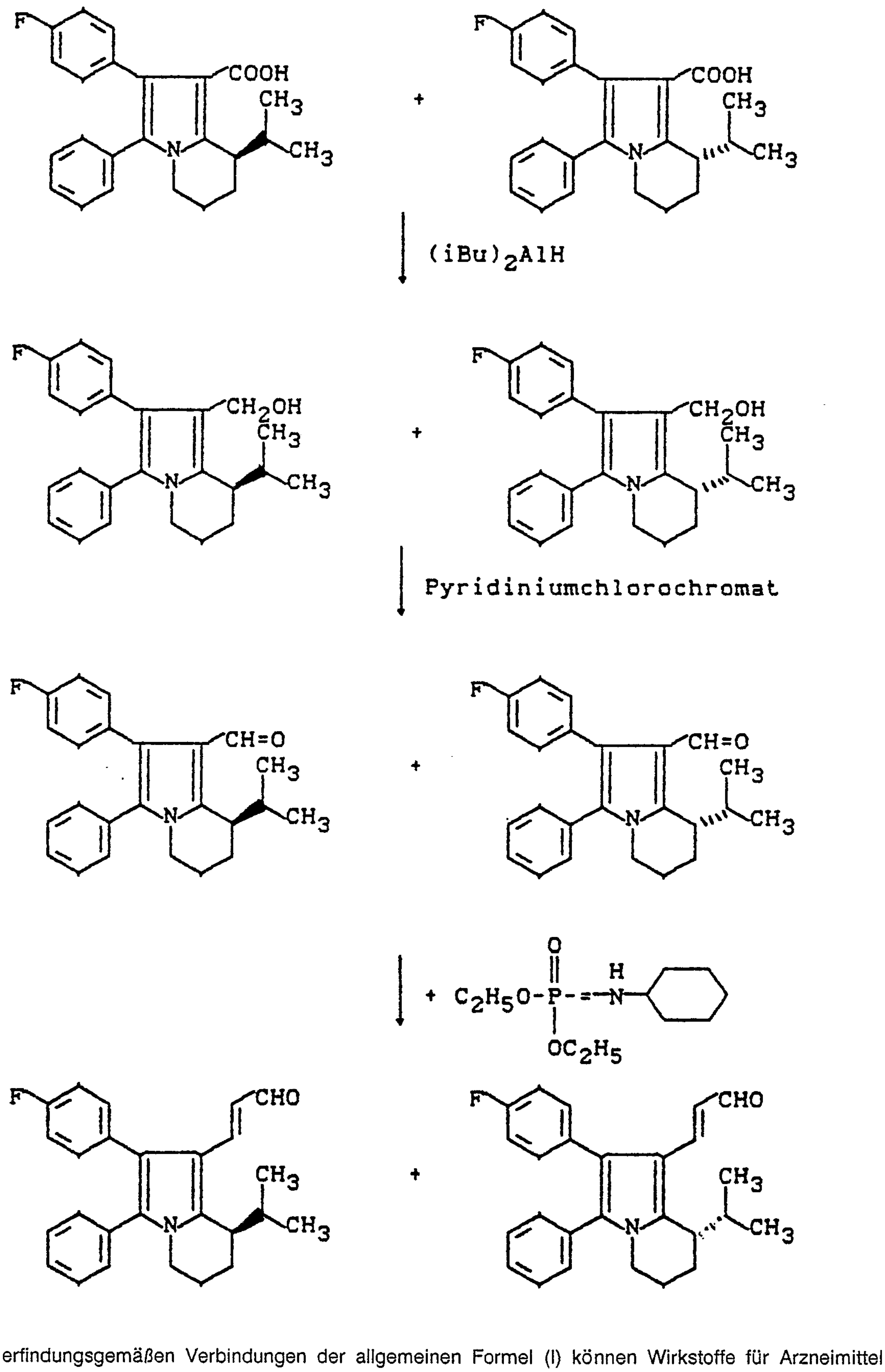


Die erfindungsgemäßen Verbindungen der allgemeinen Formel (I) können Wirkstoffe für Arzneimittel sein. Insbesondere sind sie Inhibitoren der 3-Hydroxy-3-methylglutaryl-Coenzym A (HGM-CoA) Reduktase und infolgedessen Inhibitoren der Cholesterolbiosynthese. Sie können deshalb zur Behandlung von Hyperlipoproteinämie, Lipoproteinämie oder Arteriosklerose eingesetzt werden.

Die neuen Wirkstoffe können in bekannter Weise in die üblichen Formulierungen überführt werden, wie

Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körperge wicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Herstellungsbeispiele

### Beispiel 1

(E)-4,4-Dimethyl-7-(4-fluorphenyl)-5-oxo-hept-6-ensäurenitril

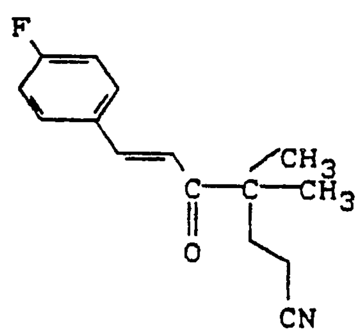

Zu einer Suspension von 124 g (1 mol) 4-Fluorbenzaldehyd und 139 g (1 mol) 4,4-Dimethyl-5-oxo-hexansäurenitril [L.B. Barkley, R.L. Levine, J. Am. Chem. Soc. 72, 3699 - 701 (1950)] in 1000 ml Wasser gibt man 120 ml 10%ige Natronlauge und rührt 60 h bei Raumtemperatur. Mit Essigsäure stellt man den pH-Wert auf 5 - 7 ein. Die organische Phase wird abgetrennt und nicht umgesetztes Ausgangsmaterial im Vakuum abdestilliert, welches erneut in die Reaktion eingesetzt und in gleicher Weise aufgearbeitet wird. Die Rückstände der Destillation werden mit Methylenchlorid über Kieselgel filtriert. Nach Einengen kristallisiert der Rückstand. Das Rohprodukt wird aus Ethanol umkristallisiert.
Ausbeute: 117 g (48% der Theorie)
Fp.: 68 - 69 °C
$^1$H-NMR ($CDCl_3$): δ - 1,3 (s, 6H, $CH_3$); 2,0 (t, 2H, $CH_2$-$CH_2$-CN); 2,3 (t, 2H, $CH_2$-CN); 7,0 (d, J = 15 Hz, 1H, Olefin-H); 7,0 - 7,2 (m, 2H, Aryl-H); 7,5 - 7,7 (m, 2H, Aryl-H); 7,7 ppm (d, J = 15 Hz, Olefin-H).

Beispiel 2

4,4-Dimethyl-5,8-dioxo-7-(4-fluorphenyl)-8-phenyl-octansäurenitril

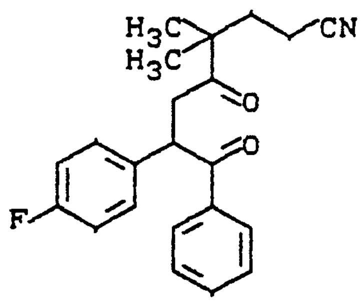

Zu einer Lösung von 5,9 g (0,12 mol) Natriumcyanid in 250 ml Dimethylformamid tropft man bei 35 °C eine Lösung von 63,6 g (0,6 mol) Benzaldehyd in 250 ml Dimethylformamid zu. Anschließend tropft man eine Lösung von 110,3 g (0,45 mol) (E)-4,4-Dimethyl-7-(4-fluorphenyl)-5-oxo-hept-6-ensäurenitril (Beispiel 1) in 250 ml Dimethylformamid zu und rührt 12 h bei Raumtemperatur. Nach Versetzen mit 1 l Wasser wird mit Methylenchlorid extrahiert. Die vereinigten organischen Phasen werden über Natriumsulfat getrocknet und eingeengt. Durch Destillation im Hochvakuum wird restliches Dimethylformamid abgetrennt. Der Rückstand wird mit Methylenchlorid über Kieselgel filtriert und eingeengt.
Ausbeute: 115,3 g (73% der Theorie) gelbes Öl
$^1$H-NMR ($CDCl_3$): δ = 1,14 und 1,22 (2s, 6H, $CH_3$); 1,8 -2,0 (m, 2H, $CH_2$-$CH_2$-CN); 2,15 - 2,30 (m, 2H, $CH_2$-CN); 2,76 (dd, 1H, CO-CH-$CH_2$-CO); 3,60 (dd, 1H, CO-CH-$CH_2$-CO); 5,12 (dd, 1H, CO-CH-$CH_2$-CO); 6,9 - 7,0 (m, 2H, Aryl-H); 7,2 - 7,5 (m, 5H, Aryl-H); 7,95 ppm (m, 2H, Aryl-H).

Beispiel 3

8,8-Dimethyl-2-(4-fluorphenyl)-3-phenyl-5,6,7,8-tetrahydroindolizin

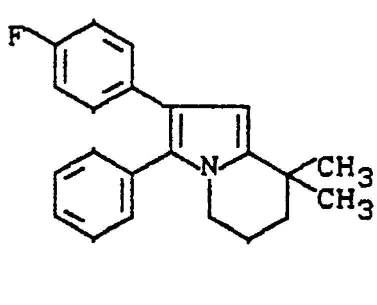

109 g (0,31 mol) 4,4-Dimethyl-5,8-dioxo-7-(4-fluorphenyl)-8-phenyl-octansäurenitril (Beispiel 2) werden in 1,2 l Methanol in Gegenwart von 15 g Raney-Kobalt bei einer Temperatur von 80 - 90 °C unter einem Druck von 50 - 60 bar Wasserstoff bis zum Ende der Wasserstoffaufnahme hydriert. Die ausgefallenen Kristalle löst man durch Zugabe von Aceton. Nach Abfiltrieren des Katalysators wird eingeengt und der Rückstand aus Ethanol umkristallisiert.
Ausbeute: 61,6 g (62% der Theorie)
Fp.: 141 - 142 °C
$^1$H-NMR ($CDCl_3$): $\delta$ = 1,38 (s, 6H, $CH_3$); 1,70 (m, 2H, $CH_2$-$C(CH_3)_2$); 1,95 (m, 2H, N-$CH_2$-$CH_2$); 3,70 (t, 2H, N-$CH_2$); 6,17 (s, 1H, Pyrrol-H); 6,75 - 6,90 (m, 2H, Aryl-H); 7,05 - 7,15 (m, 2H, Aryl-H); 7,25 - 7,40 ppm (m, 5H, Aryl-H).

Beispiel 4

(E)-3-[8,8-Dimethyl-2-(4-fluorphenyl)-3-phenyl-5,6,7,8-tetrahydroindolizin-1-yl]-prop-2-enal

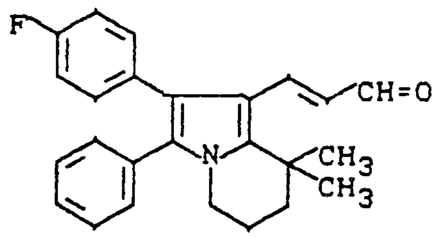

Zu einer Lösung von 13,8 g (90 mmol) Phosphoroxychlorid in 200 ml wasserfreiem Acetonitril tropft man unter Stickstoff bei -10 °C, 9,8 g (90 mmol) 90%iges Dimethylaminoacrolein in 50 ml Acetonitril zu. Bei 0 °C gibt man 19,15 g (60 mmol) 8,8-Dimethyl-2-(4-fluorphenyl)-3-phenyl-5,6,7,8-tetrahydroindolizin (Beispiel 3) zu. Man läßt auf Raumtemperatur kommen und rührt anschließend 24 h bei 40 °C. Den Ansatz gibt man nun bei 10 °C in eine Suspension aus 22 g Natriumhydroxid in 450 ml Wasser und 450 ml Toluol. Nach 1h Rühren trennt man die Toluolphase ab. Nach Waschen mit Wasser, Trocknen über Natriumsulfat und Einengen kristallisiert man aus Ethanol um.
Ausbeute: 13,7 g (61% der Theorie)
Fp.: 182 - 183 °C
$^1$H-NMR ($CDCl_3$); $\delta$ = 1,58 (s, 6H, $CH_3$); 1,75 - 1,85 (m, 2H, $CH_2$-$C(CH_3)_2$); 1,85 - 2,00 (m, 2H, N-$CH_2$-$CH_2$); 3,72 (t, 2H, N-$CH_2$); 5,63 (dd, 1H, Olefin-H); 6,80 - 6,95 (m, 2H, Aryl-H); 7,00 - 7,30 (m, 7H, Aryl-H); 7,80 (d, 1H, Olefin-H); 9,38 ppm (d, 1H, CHO).

Beispiel 5

Methyl-(E)-7-[8,8-dimethyl-2-(4-fluorphenyl)-3-phenyl-5,6,7,8-tetrahydroindolizin-1-yl]-5-hydroxy-3-oxo-hept-6-enoat

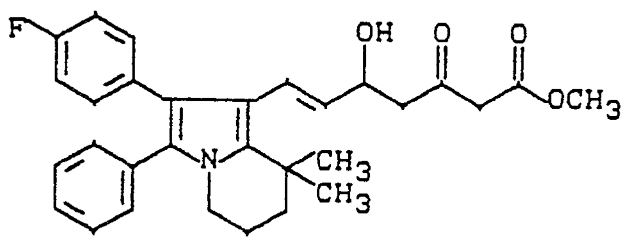

Unter Stickstoff tropft man zu einer Suspension von 0,96 g (32 mmol) 80%iges Natriumhydrid in 50 ml wasserfreiem Tetrahydrofuran bei 0 °C 3,0 g (30 mmol) Acetessigsäuremethylester in 20 ml Tetrahydrofuran. Nach 15 min werden bei derselben Temperatur 14,4 ml (36 mmol) einer 2,5 molaren Lösung von n-Butyllithium in Hexan zugetropft. Nach 15 min werden bei derselben Temperatur 7,46 g (20 mmol) (E)-3-[8,8-Dimethyl-2-(4-fluorphenyl)-3-phenyl-5,6,7,8-tetrahydroindolizin-1-yl]-prop-2-enal (Beispiel 4) in 100 ml Tetrahydrofuran gelöst zugetropft. Nach 15 Minuten tropft man 4 g Eisessig in 40 ml Wasser zu. Der Ansatz wird auf Eiswasser gegossen und nach Abtrennung der organischen Phase zweimal mit Ether extrahiert. Die vereinigten organischen Phasen werden zweimal mit Natriumhydrogencarbonatlösung und zweimal mit gesättigter Kochsalzlösung gewaschen. Nach Trocknen über Natriumsulfat und Einengen chromatographiert und mit Cyclohexan/Aceton (2:1) an 250 g Kieselgel (70-230 mesh). Ausbeute: 8,9 g (91% der Theorie) rötliches Öl

$R_f$ = 0,28

$^1$H-NMR (DMSO): δ = 1,38 (s, 6H, $CH_3$); 1,65 - 1,75 (m, 2H, $CH_2$-$C(CH_3)_2$); 1,75 - 1,90 (m, 2H, N-$CH_2$-$CH_2$); 2,40 (m, 2H, CH(OH)-$CH_2$); 3,60 (s, 3H, $COOCH_3$); 3,65 (m, 2H, N-$CH_2$); 4,35 (m, 1H, CH(OH); 4,85 (d, 1H, CH-$COOCH_3$ (Enolform)); 4,90 (dd, 1H, Olefin-H); 6,65 (d, 1H, Olefin-H); 6,9 - 7,3 ppm (m, 9H, Aryl-H).

Beispiel 6

Methyl-erythro-(E)-3,5-dihydroxy-7-[8,8-dimethyl-2-(4-fluorphenyl)-3-phenyl-5,6,7,8-tetrahydroindolizin-1-yl]-hept-6-enoat

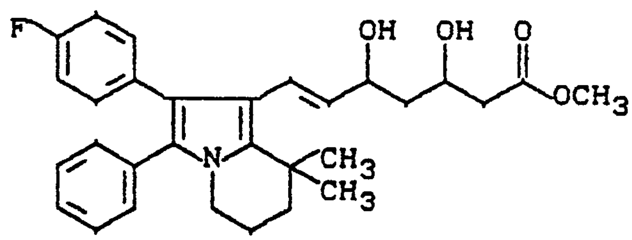

Unter Stickstoff tropft man zu einer Lösung von 4,89 g (10 mmol) Methyl-(E)-7-[8,8-dimethyl-2-(4-fluorphenyl)-3-phenyl-5,6,7,8-tetrahydroindolizin-1-yl]-5-hydroxy-3-oxo-hept-6-enoat (Beispiel 5) in 50 ml wasserfreiem Tetrahydrofuran 12 ml (12 mmol) einer 1 molaren Lösung von Triethylboran in Hexan bei Raumtemperatur zu, leitet während 5 min Luft durch die Lösung und kühlt dann auf -80 °C ab. Man gibt 0,45 g (12 mmol) Natriumborhydrid zu und tropft langsam 6,7 ml Methanol zu, rührt 15 min bei -75 °C und 15 min bei -30 °C. Anschließend tropft man eine Lösung von 32,5 ml 30%igem Wasserstoffperoxid in 65 ml Wasser zu. Im Verlauf der Zugabe läßt man die Temperatur auf Raumtemperatur steigen. Nach Phasentrennung extrahiert man mit Essigester. Die vereinigten organischen Phasen werden zweimal mit Natriumhydrogencarbonatlösung und zweimal mit gesättigter Kochsalzlösung gewaschen. Nach Trocknen über Natriumsulfat und Einengen chromatographiert man mit Cyclohexan/Aceton (2:1) an 250 g Kieselgel (35-70 µm).

Ausbeute: 3,5 g (71% der Theorie) amorpher Feststoff

$R_f$ = 0,20

$^1$H-NMR (DMSO): δ = 1,38 (s, 6H, $CH_3$); 1,65 - 1,75 (m, 2H, $CH_2$-$C(CH_3)_2$); 1,75 - 1,90 (m, 2H, N-$CH_2$-$CH_2$); 2,20 - 2,45 (2 dd, 2H, $CH_2$-$COOCH_3$); 3,55 (s, 3H, $COOCH_3$); 3,65 (m, 2H, N-$CH_2$); 3,80 und 4,05 (2m, 2H, CH(OH)); 4,88 (dd, 1H, Olefin-H); 6,60 (d, 1H, Olefin-H); 6,9 - 7,3 ppm (m, 9H, Aryl-H).

Beispiel 7

(E)-7-(4-Fluorphenyl)-5-oxo-4-(2-propyl)-hept-6-ensäurenitril

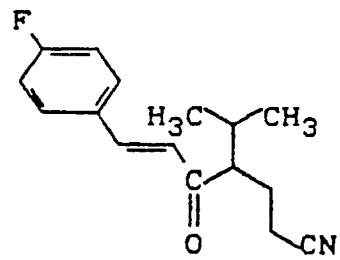

Zu einer Lösung von 4,2 g (0,105 mol) Natriumhydroxid in 100 ml Wasser gibt man eine Lösung von 34,9 g (0.228 mol) 5-Oxo-4-(2-propyl)-hexansäurenitril [G. Näslund, A. Senning, S.O. Lawesson; Acta Chem. Scand. 16, 1324 - 8 (1962)] und 28,3 g (0,228 mol) 4-Fluorbenzaldehyd in 400 ml Methanol und rührt 96 h bei Raumtemperatur. Nach Einengen wird mit Wasser versetzt, mit Methylenchlorid extrahiert, über Natriumsulfat getrocknet und eingeengt. Der Rückstand wird mit Methylenchlorid über Kieselgel filtriert.
Ausbeute: 31,7 g (54% der Theorie)
$^1$H-NMR ($CDCl_3$): $\delta$ = 0,90 und 1,01 (2d, 6H, $CH_3$); 1,8 (m, 1H, $CH(CH_3)_2$; 2,0 - 2,5 (m, 4H, $CH_2$-$CH_2$-CN); 2,85 (m, 1H, CO-CH); 6,77 (d, J = 15 Hz, 1H, Olefin-H); 7,0 - 7,2 (m, 2H, Aryl-H); 7,5 - 7,7 ppm (m, 3H, 2 Aryl-H und 1 Olefin-H).

Beispiel 8

5,8-Dioxo-7-(4-fluorphenyl)-8-phenyl-4-(2-propyl)-octansäurenitril

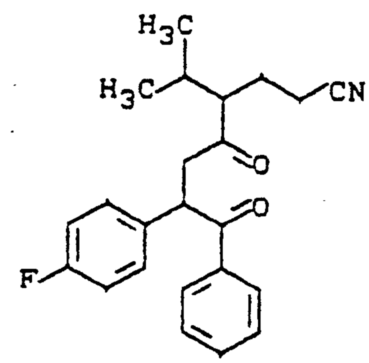

Zu einer Lösung von 1,5 g (0,031 mol) Natriumcyanid in 65 ml Dimethylformamid tropft man bei 35°C eine Lösung von 17,0 g (0,16 mol) Benzaldehyd in 65 ml Dimethylformamid zu. Anschließend tropft man eine Lösung von 31,1 g (0,12 mol) (E)-7-(4-Fluorphenyl)-5-oxo-4-(2-propyl)-hept-6-ensäurenitril (Beispiel 7) in 65 ml Dimethylformamid zu und rührt 12 h bei Raumtemperatur. Nach Versetzen mit 250 ml Wasser wird mit Methylenchlorid extrahiert. Die vereinigten organischen Phasen werden über Natriumsulfat getrocknet und eingeengt. Durch Destillation im Hochvakuum wird restliches Dimethylformamid abgetrennt. Der Rückstand wird mit Methylenchlorid über Kieselgel filtriert und eingeengt.
Ausbeute: 33.2 g (76% der Theorie) gelbes Öl.
$^1$H-NMR ($CDCl_3$): $\delta$ = 0,80; 0.88; 0,94 und 1,01 (4d, 6H, $CH_3$); 1,60 - 1,85 (m, 1H, $CH(CH_3)_2$); 1,85 - 2,45 (m, 4H, $CH_2$-$CH_2$-CN); 2,45 - 2,75 (2m, 1H, CO-CH-$CH_2$-$CH_2$-CN); 2,65 - 2,90 (m, 1H, $CH_2$-CO); 3,50 und 3,72 (2dd, 1H, $CH_2$-CO); 5,12 (dd, 1H, CO-CH-$CH_2$-CO); 6,95 (m, 2H, Aryl-H); 7,15 - 7,60 (m, 5H, Aryl-H): 7,95 ppm (m, 2H, Aryl-H).

Beispiel 9

2-(4-Fluorphenyl)-3-phenyl-8-(2-propyl)-5,6,7,8,-tetrahydroindolizin

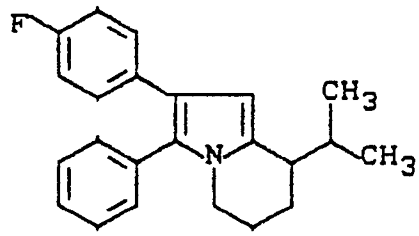

32,4 g (89 mmol) 5,8-Dioxo-7-(4-fluorphenyl)-8-phenyl-4-(2-propyl)-octansäurenitril (Beispiel 8) werden in 400 ml Methanol in Gegenwart von 5 g Raney-Kobalt bei einer Temperatur von 80-90° C unter einem Druck von 50-60 bar Wasserstoff bis zum Ende der Wasserstoffaufnahme hydriert. Die ausgefallenen Kristalle löst man durch Zugabe von Aceton. Nach Abfiltrieren des Katalysators wird eingeengt und der Rückstand aus Ethanol umkristallisiert.
Ausbeute: 24,8 g (83% der Theorie)
Fp.: 112 - 114° C
$^1$H-NMR ($CDCl_3$): $\delta$ = 0.92 und 1,07 (2d, 6H, $CH_3$); 1,45 - 1,55 (m, 1H, $CH_2$-CH-CH$(CH_3)_2$); 1,65 - 2,10 (m, 3H, $CH_2$-$CH_2$-CH-CH$(CH_3)_2$); 2,30 (m, 1H, CH$(CH_3)_2$; 2,85 (m, 1H, CH-CH$(CH_3)_2$); 3,55 -3,85 (m, 2H, N-$CH_2$); 6,15 (s, 1H, Pyrrol-H); 6,85 (m, 2H, Aryl-H); 7,10 (m, 2H, Aryl-H); 7,30 ppm (m, 5H, Aryl-H).

Beispiel 10

(E)-3-[2-(4-Fluorphenyl)-3-phenyl-8-(2-propyl)-5,6,7,8-tetrahydroindolizin-1-yl]-prop-2-enal

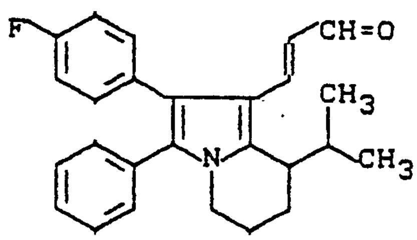

Zu einer Lösung von 9,2 g (60 mmol) Phosphoroxychlorid in 65 ml wasserfreiem Acetonitril tropft man unter Stickstoff bei -10° C 6,6 g (60 mmol) 90%iges Dimethylaminoacrolein in 35 ml Acetonitril zu. Bei 0° C gibt man 15,5 g (46,5 mmol) 2-(4-Fluorphenyl)-3-phenyl-8-(2-propyl)-5,6,7,8-tetrahydroindolizin (Beispiel 9) zu. Man läßt auf Raumtemperatur kommen und rührt anschließend 20 h bei dieser Temperatur. Den Ansatz gibt man nun unter kräftigem Rühren zu einer eisgekühlten Lösung von 16 g Natriumhydroxid in 300 ml Wasser. Der ausgefallene Feststoff wird gründlich mit Wasser alkalifrei gewaschen und aus Ethanol Umkristallisiert.
Ausbeute: 14,5 g (80 % der Theorie)
Fp.: 164 - 165° C
$^1$H-NMR ($CDCl_3$): $\delta$ = 0,88 und 1,04 (2d, 6H, $CH_3$); 1,65 - 2,35 (m, 5H, N-$CH_2$-$CH_2$-$CH_2$-CH-CH$(CH_3)_2$); 3,25 (q, 1H, CH-CH$(CH_3)_2$); 3,6 - 3,9 (m, 2H, N-$CH_2$); 5,93 (dd, 1H, Olefin-H); 6,85 -7,30 (m, 9H, Aryl-H); 7,42 (d, 1H, Olefin-H); 9,40 ppm (d, 1H, CHO).

Beispiel 11

Methyl-(E)-7-[2-(4-fluorphenyl)-3-phenyl-8-(2-propyl)-5,6,7,8-tetrahydroindolizin-1-yl]-5-hydroxy-3-oxo-hept-6-enoat

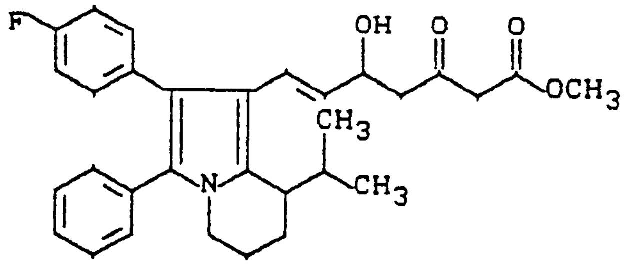

Unter Stickstoff tropft man zu einer Suspension von 0,96 g (32 mmol) 80%igem Natriumhydrid in 50 ml Wasserfreiem Tetrahydrofuran bei 0 °C 3,0 g (30 mmol) Acetessigsäuremethylester in 20 ml Tetrahydrofuran. Nach 15 min. werden bei derselben Temperatur 14,4 ml (36 mmol) eine 2,5 molaren Lösung von n-Butyllithium in Hexan zugetropft. Nach 15 min werden bei derselben Temperatur 7,74 g (20 mmol) (E)-3-[2-(4-Fluorphenyl)-3-phenyl-8-(2-propyl)- 5,6,7,8-tetrahydroindolizin-1-yl]-prop-2-enal (Beispiel 10) in 100 ml Tetrahydrofuran gelöst zugetropft. Nach 15 min tropft man 4 g Eisessig in 40 ml Wasser zu. Der Ansatz wird auf Eiswasser gegossen und nach Abtrennung der organischen Phase zweimal mit Ether extrahiert. Die vereinigten organischen Phasen werden zweimal mit Natriumhydrogencarbonatlösung und zweimal mit gesättigter Kochsalzlösung gewaschen. Nach Trocknen über Natriumsulfat wird eingeengt. Das Rohprodukt wird ohne weitere Reinigung weiterverwendet.
Ausbeute: 10,6 g rötliches Öl,
$R_f$ = 0,32 (Cyclohexan/Aceton 2:1)

Beispiel 12

Methyl-erythro-(E)-3,5-dihydroxy-7-[2-(4-fluorphenyl)-3-phenyl-8-(2-propyl)-5,6,7,8-tetrahydroindolizin-1-yl]-hept-6-enoat

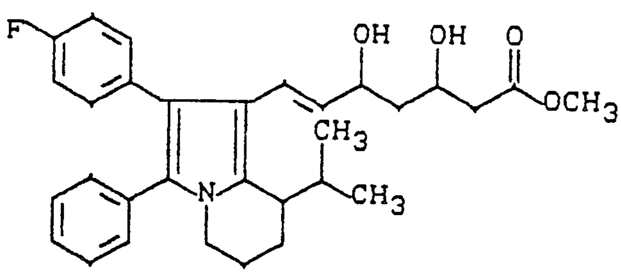

Unter Stickstoff tropft man zu einer Lösung von 10,6 g (20 mmol) Methyl-(E)-7-[2-(4-fluorphenyl)-3-phenyl)-8-(2-propyl)-5,6,7,8-tetrahydroindolizin-1-yl]-5-hydroxy-3-oxo-hept-6-enoat (Beispiel 11) in 100 ml wasserfreiem Tetrahydrofuran 24 ml (24 mmol) einer 1 molaren Lösung von Triethylboran in Hexan bei Raumtemperatur zu, leitet während 5 min Luft durch die Lösung und kühlt dann auf -80 °C ab. Man gibt 0,9 g (24 mmol) Natriumborhydrid zu und tropft langsam 13,4 ml Methanol zu, rührt 15 min bei -75 °C und 15 min bei -30 °C. Anschließend tropft man eine Lösung von 65 ml 30%igem Wasserstoffperoxid in 130 ml Wasser zu. Im Verlauf der Zugabe läßt man die Temperatur auf Raumtemperatur steigen. Nach Phasentrennung extrahiert man mit Essigester. Die vereinigten organischen Phasen werden zweimal mit Natriumhydrogencarbonatlösung und zweimal mit gesättigter Kochsalzlösung gewaschen. Nach Trocknen über Natriumsulfat und Einengen chromatographiert man mit Cyclohexan/Aceton (2:1) an 250 g Kieselgel (35-70 µm).
Ausbeute: 6,4 g (63% der Theorie, über 2 Stufen), amorpher Feststoff.
$R_f$ = 0,27
$^1$H-NMR (DMSO): $\delta$ = 0,79 (dd, 3H, $CH_3$); 0,95 (d, 3H, $CH_3$); 1,5 - 2,1 (m, 5H, N-$CH_2$-$CH_2$-$CH_2$-CH-CH-$(CH_3)_2$); 2,20 - 2,45 (m, 2H, $CH_2$-$COOCH_3$); 3,55 (s, 3H, $COOCH_3$); 3,65 - 4,15 (m, 4H, N-$CH_2$ und 2 CH-(OH)); 5,10 - 5,35 (m, 1H, Olefin-H); 6,28 (dd, 1H, Olefin-H); 6,9 - 7,3 ppm (m, 9H, Aryl-H).

Beispiel 13

2-Acetyl-5-cyano-2-ethyl-pentansäure-tert.butylester

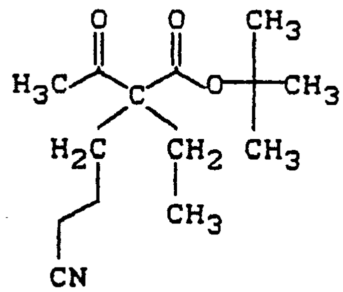

Zu einer Suspension von 21,5 g (0,715 mol) 80%igem Natriumhydrid in 450 ml wasserfreiem Dioxan tropft man unter Stickstoff 121 g (0,65 mol) 2-Ethyl-3-oxo-butansäure-tert.butylester zu. Anschließend tropft man bei einer Temperatur von 60 °C 105,8 g (0,715 mol) 4-Brom-buttersäurenitril zu und erhitzt 48 h zum Rückfluß. Nach abkühlung tropft man 50 ml Methanol zu, saugt vom ausgefallenen Natriumbromid ab und engt ein. Der Rückstand wird mit Methylenchlorid über Kieselgel filtriert.
Ausbeute: 104,3 g (63% der Theorie)
$^1$H-NMR ($CDCl_3$): δ = 0,78 (t, 3H, $CH_2$-$\underline{CH_3}$); 1,45 (s, 9H, $OC(CH_3)_3$); 1,8 - 2,0 (m, 6H, $\underline{CH_2}$-$\underline{CH_2}$-$CH_2$-CN, $\underline{CH_2}$-$CH_3$); 2,13 (s, 3H, $CH_3CO$); 2,36 ppm (t, 2H, $CH_2$-CN).

Beispiel 14

4-Ethyl-5-oxo-heptansäurenitril.

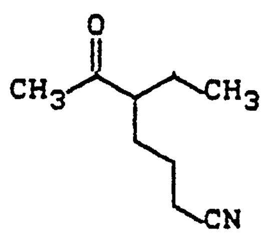

101,2 g (0,4 mol) 2-Acetyl-5-cyano-2-ethyl-pentansäuretert.-butylester werden zusammen mit 0,4 g 4-Toluolsulfonsäure in einer Destillationsapparatur vorgelegt und langsam bis auf 150 °C erhitzt. Man hält diese Temperatur bis zum Ende der Gasentwicklung. Anschließend wird im Vakuum destilliert.
Ausbeute: 41,1 g (67 % der Theorie)
Kp: = 130-135 °C/4 mbar
$^1$H-NMR ($CDCl_3$): δ = 0,89 (t, 3H, $CH_2$-$\underline{CH_3}$); 1,5 - 1,8 (m, 6H, $\underline{CH_2}$-$\underline{CH_2}$-$CH_2$-CN, $\underline{CH_2}$-$CH_3$); 2,13 (s, 3H, $CH_3$-CO); 2,36 (t, 2H, $CH_2$-CN); 2,57 ppm (m 1H, CO-CH).

Beispiel 15

(E)-5-Ethyl-8-(4-fluorphenyl)-6-oxo-oct-7-ensäurenitril

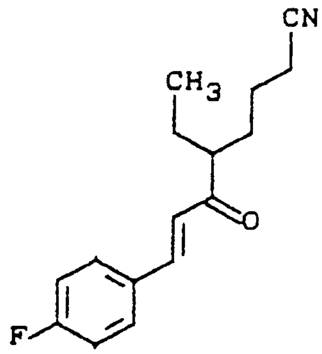

Analog Beispiel 7 erhält man aus 40,3 g (0,263 mol) 4-Ethyl-5-oxo-heptansäurenitril und 32,6 g (0,263 mol) 4-Fluorbenzaldehyd die Titelverbindung.
Ausbeute: 39,4 g (58% der Theorie)
$^1$H-NMR ($CDCl_3$): δ = 0,90 (t, 3H, $CH_3$); 1,5 - 1,9 (m, 6H, $CH_2$-$CH_2$-$CH_2$-CN, $CH_2$-$CH_3$); 2,35 (t, 2H, $CH_2$-CN); 2,75 (m, 1H, CO-CH); 6,75 (d, 1H, Olefin-H); 7,0 - 7,2 (m, 2H, Aryl-H); 7,5 - 7,7 ppm (m, 3H, 2 Aryl-H und 1 Olefin-H).

Beispiel 16

6,9-Dioxo-5-ethyl-8-(4-fluorphenyl)-9-phenyl-nonansäurenitril

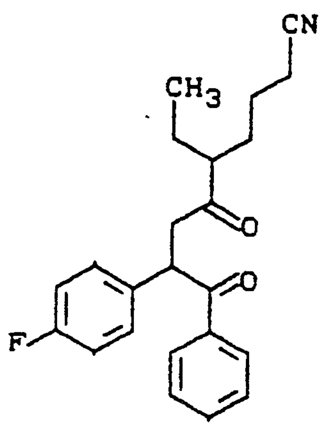

Analog Beispiel 8 erhält man aus 39 g (0,15 mol) (E)-5-Ethyl-8-(4-fluorphenyl)-6-oxo-oct-7-ensäurenitril (Beispiel 15) und 21,2 g (0,2 mol) Benzaldehyd die Titelverbindung.
Ausbeute: 40,9 g (75% der Theorie)
$^1$H-NMR ($CDCl_3$): δ = 0,81 und 0,88 (2t, 3H, $CH_3$); 1,3 -1,9 (m, 6H, $CH_2$-$CH_2$-$CH_2$-CN, $CH_2$-$CH_3$); 2,3 (m, 2H, $CH_2$CN); 2,45 und 2,55 (2 m, 1H, CO-CH); 2,65 - 2,85 (2 dd, 1H, CO-CH-$CH_2$-CO); 3,40 -3,70 (2dd, 1H, CO-CH-$CH_2$-CO); 6,95 (m, 2H, Aryl-H); 7,15 - 7,60 (m, 5H, Aryl-H); 7,95 ppm (m, 2H, Aryl-H).

Beispiel 17

9-Ethyl-2-(4-fluorphenyl)-3-phenyl-5,6,7,8-tetrahydro-9H-pyrrolo-[1,2-a]-azepin

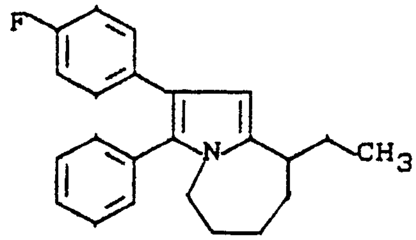

Analog Beispiel 9 erhält man aus 40 g (0,109 mol) 6,9 Dioxo-5-ethyl-8-(4-fluorphenyl)-9-phenylnonansäurenitril (Beispiel 16) die Titelverbndung.
Ausbeute: 20,4 g (56% der Theorie)
Fp.: 142 - 143 °C
$^1$H-NMR ($CDCl_3$): $\delta$ = 1,09 (t, 3H, $CH_3$); 1,25 - 2,15 (m 8H, C-$CH_2$-C); 2,60 (m, 1H, CH-$CH_2$-$CH_3$); 3,65 und 3,97 (m, 2H, N-$CH_2$); 6,06 (s, 1H, Pyrrol-H); 6,79 (t, 2H, Aryl-H); 6,95 - 7,45 ppm (m, 7H, Aryl-H).

## Beispiel 18

(E)-3-[9-Ethyl-2-(4-fluorphenyl)-3-phenyl-5,6,7,8-tetrahydro-9H-pyrrolo-[1,2-a]-azepin-1-yl]-prop-2-enal

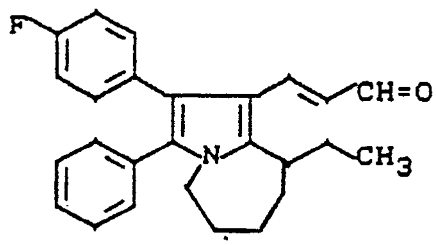

Analog Beispiel 4 erhält man aus 20 g (60 mmol) 9-Ethyl-2-(4-fluorphenyl)-3-phenyl-5,6,7,8-tetrahydro-9H-pyrrolo-[1,2-a]-azepin (Beispiel 17) die Titelverbindung.
Ausbeute: 13,7 g (59% der Theorie:
Fp.: 138 - 139 °C
$^1$H-NMR ($CDCl_3$): $\delta$ = 1,00 (t, 3H, $CH_3$); 1,5 - 2,2 (m, 8H, C-$CH_2$-C); 3,40 (m, 1H, CH-$CH_2$-$CH_3$); 3,72 und 4,08 (2 dd, 2H, N-$CH_2$); 5,78 (dd, 1H, Olefin-H); 6,88 (t, 2H, Aryl-H); 7,05 (m, 5H, Aryl-H); 7,25 (m, 2H, Aryl-H); 7,45 (d, 1H, Olefin-H); 9,40 ppm (d, 1H, CHO).

## Beispiel 19

Methyl-(E)-7-[9-Ethyl-2-(4-fluorphenyl)-3-phenyl-5,6,7,8-tetrahydro-9H-pyrrolo[1,2-a]-azepin-1-yl]-5-hydroxy-3-oxo-hept-6-enoat

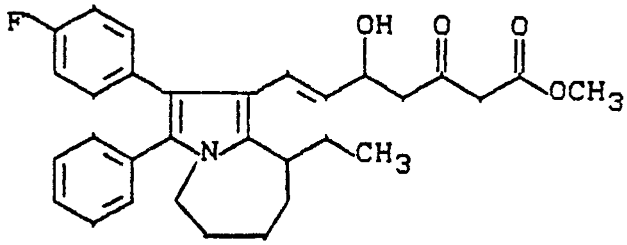

Analog Beispiel 11 erhält man aus 3,87 g (10 mmol) (E)-3-[9-Ethyl-2-(4-fluorphenyl)-3-phenyl-5,6,7,8-tetrahydro-9H-pyrrolo-[1,2-a]-azepin-1-yl]-prop-2-enal (Beispiel 18) die Titelverbindung.
Ausbeute: 5,0 g rötliches Öl
$R_f$ = 0,26 (Cyclohexan/Aceton 2:1)

Beispiel 20

Methyl-erythro-(E)-3,5-dihydroxy-7-[9-ethyl-2-(4-fluorphenyl)-3-phenyl-5,6,7,8-tetrahydro-9H-pyrrolo-[1,2-a]-azepin-1-yl]-hept-6-enoat

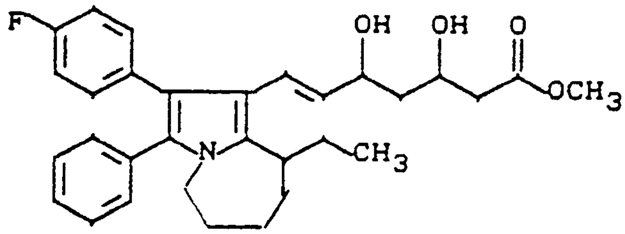

Analog Beispiel 12 erhält man aus 5,0 g (10 mmol) Methyl-erythro-(E)-7-[9-ethyl-2-(4-fluorphenyl)-3-phenyl-5,6,7,8-tetrahydro-9H-pyrrolo-[1,2-a]-azepin-1-yl]-5-hydroxy-3-oxo-hept-6-enoat (Beispiel 19) die Titelverbindung.
Ausbeute: 3,2 g (65% der Theorie, über 2 Stufen) amorpher Feststoff
$R_f$ = 0.19 (Cyclohexan / Aceton 2:1)
$^1$H-NMR (DMSO) : $\delta$ = 0.94 (m, 3H, $CH_3$); 1,3 - 2,1 (m, 8H, C-$CH_2$-C); 2,25 - 2,45 (m, 2H, $CH_2$-$COOCH_3$); 3,58 (s, 3H, $COOCH_3$); 3,60 - 4,15 (m, 4H, N-$CH_2$ und 2 CH(OH)); 5,20 (ddd, 1H, Olefin-H); 6,28 (d, 1H, Olefin-H); 6,90 - 7,35 ppm (m, 9H, Aryl-H).

Anwendungsbeispiele

Beispiel 21

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und im Potter-Elvejem-Homogenisator 3 mal in 3 Volumen 0,1 m Saccharose, 0,05 m KCl, 0,04 m $K_xH_y$ Phosphat, 0,03 m Ethylendiamintetraessigsäure, 0,002 m Dithiothreit (SPE)-Puffer pH 7,2, homogenisiert. Anschließend wurde 15 Minuten bis 15 000* g zentrifugiert und das Sediment verworfen. Der Überstand wurde 75 Minuten bei 100 000 g sedimentiert. Das Pellet wurde in 1/4 Volumen SPE-Puffer aufgenommen, nochmals homogenisiert und anschließend erneut 60 Minuten bei 100.000 g zentrifugiert. Das Pellet wurde mit der 5-fachen Menge ihres Volumes SPE-Puffer aufgenommen, homogenisiert und bei -78°C eingefroren und gelagert (= Enzymlösung).

Zur Testung wurden die Testverbindungen (oder Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindun gen mit dem Enzym bei 37°C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase, 35 µMol $K_xH_y$-Phosphat PH 7,2, 20 µl Enzympräparation und 56 nMol 3-Hydroxy-3-methyl-glutaryl Coenzym A (Glutaryl-3-$^{14}$C) 100 000 dpm.

Nach Inkubation von 60 Minuten bei 37°C wurde der Ansatz zentrifugiert und 600 µl des Überstandes auf eine 0,7 x 4 cm mit einem 5-Chlorid 100-200 mesh (Anionenaustauscher) gefüllte Säule aufgetragen. Es wurde mit 2 ml dest. Wasser nachgewaschen und Durchlauf plus Waschwasser mit 3 ml Aquasol versetzt

und im LKB-Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 100 gesetzt und mit dem simultan bestimmten $IC_{50}$-Wert der Testverbindung verglichen.

| Beispiel-Nr. | relative $IC_{50}$-Werte |
|---|---|
| 6 | 37 |
| 12 | 77 |
| 20 | 25 |

Beispiel 22

Die Serum-Cholesterin-senkende Wirkung der erfindungsgemäßen Verbindungen auf die Blutcholesterinwerte von Hunden wurde in mehrwöchigen Fütterungsexperimenten gefunden. Dazu wurde die zu untersuchende Substanz über einen mehrwöchigen Zeitraum einmal täglich in einer Kapsel gesunden Beagle-Hunden zusammen mit dem Futter p.o. gegeben. Dem Futter war außerdem während der gesamten Versuchsperiode, d.h. vor, während und nach der Applikationsperiode der zu untersuchenden Substanz Cholestryamin (4 g/100 g Futter) als Gallensäuresequestrant beigemischt.

Zweimal wöchentlich wurde den Hunden venöses Blut abgenommen und das Serumcholesterin mit einem handelsüblichen Testkit enzymatisch bestimmt. Die Serumcholesterinwerte während der Applikationsperiode wurden mit den Serumcholesterinwerten vor der Applikationsperiode (Kontrolle) verglichen.

**Ansprüche**

1. Substituierte anellierte Pyrrole der allgemeinen Formel (I)

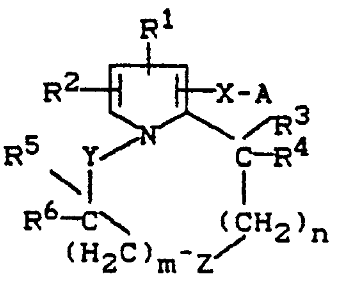

(I)

in welcher

$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$, $R^8$ - gleich oder verschieden sind und für Alkyl, Aryl, Aralkyl, Acyl, Alkylsulfonyl oder Arylsulfonyl stehen,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben,

$R^2$ - Heteroaryl bedeutet, das bis zu 3-fach gleich oder verschieden durch Halogen, Alkyl, Alkoxy, Alkylthio,

Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,
worin
$R^7$, $R^8$ - die oben angegebene Bedeutung haben,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,
oder
- Cycloalkyl bedeutet, oder
- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,
worin
$R^7$, $R^8$ - die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^3$, $R^4$, $R^5$, $R^6$ gleich oder verschieden sind und
- Wasserstoff bedeuten, oder
- Cycloalkyl bedeuten, oder
- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,
worin
$R^7$, $R^8$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
- Heteroaryl bedeuten, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,
worin
$R^7$ und $R^8$ die oben angegebene Bedeutung haben,
oder
- Aryl bedeuten, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,
oder $R^3$ und $R^4$ und/oder $R^5$ und $R^6$ jeweils gemeinsam einen Ring bilden, der gegebenenfalls durch ein Sauerstoffatom, ein Schwefelatom, oder eine Gruppe der Formel $-NR^9$ unterbrochen sein kann,
worin
$R^9$ - für Wasserstoff, Alkyl, Aryl, Carbamoyl, Alkoxycarbonyl steht,
X - eine Gruppe der Formel $-CH_2-CH_2-$ oder $-CH=CH-$bedeutet,
A - eine Gruppe der Formel

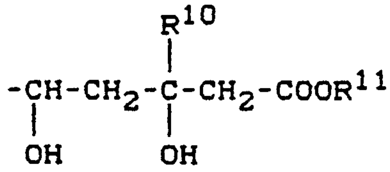 oder 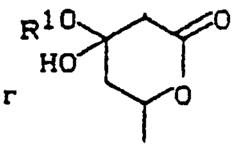

bedeutet.
worin
$R^{10}$ - für Wasserstoff oder Alkyl steht,
und
$R^{11}$ - für Wasserstoff steht, oder
- für Alkyl, Aralkyl oder Aryl steht, oder
- für ein Kation steht,
Y - eine Bindung bedeutet, oder
- eine Gruppe der Formel $-NR^{12}$ bedeutet
worin
$R^{12}$ - für Wasserstoff oder Alkyl steht,
Z - eine Gruppe der Formel $-CH=CH-$ oder $-CH_2-$bedeutet, oder
ein Schwefelatom, Sauerstoffatom, oder
- für eine Gruppe der Formel $-NR^{12}$ bedeutet,
worin
$R^{12}$ die oben angegebene Bedeutung hat,
und
n und m gleich oder verschieden sind und
- eine Zahl 0, 1, 2, 3 oder 4 bedeuten.

2. Substituierte anellierte Pyrrole nach Anspruch 1
worin
$R^1$ - Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder ver schieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^7R^8$,
wobei
$R^7$ und $R^8$ gleich oder verschieden sind und Niederalkyl, Phenyl, Benzyl, Acetyl, Benzoyl, Phenylsulfonyl oder Niederalkylsulfonyl bedeuten,
$R^2$ - Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder ver schieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^7R^8$,
wobei
$R^7$ und $R^8$ die oben angegebene Bedeutung haben,
- oder Cyclopropyl, Cyclopentyl oder Cyclohexyl bedeutet, oder
- Niederalkyl bedeutet, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^7R^8$,
worin

$R^7$ und $R^8$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,
$R^3$, $R^4$, $R^5$, $R^6$ gleich oder verschieden sein können, und
- Wasserstoff, oder
- Cyclopropyl, Cyclopentyl oder Cyclohexyl bedeuten, oder
- Niederalkyl bedeuten, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^7R^8$,
worin
$R^7$ und $R^8$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, Niederalkyl, Niederalkoxy, Trifluormethyl, oder Trifluormethoxy substituiert sein können, oder
- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeuten, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeuten, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^7R^8$,
wobei
$R^7$ und $R^8$ die oben angegebene Bedeutung haben,
oder
$R^3$ und $R^4$ und/oder $R^5$ und $R^6$ jeweils gemeinsam einen Ring bilden, der gegebenenfalls durch ein Sauerstoffatom, ein Schwefelatom oder durch eine Gruppe der Formel $-NR^9$ unterbrochen sein kann,
worin
$R^9$ - für Wasserstoff, Niederalkyl, Phenyl, Benzyl oder Niederalkoxycarbonyl steht,
X - eine Gruppe der Formel $-CH=CH-$ bedeutet
A - eine Gruppe der Formel

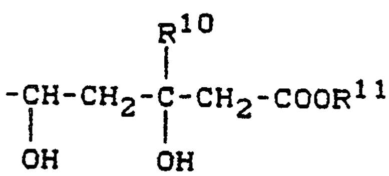

oder

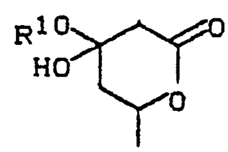

bedeutet,
worin
$R^{10}$ - für Wasserstoff oder Niederalkyl steht,
$R^{11}$ - für Wasserstoff steht, oder
- für Niederalkyl oder Phenyl steht, oder
- für ein physiologisch verträgliches Kation steht, oder
- für Benzyl steht,
Y - eine Bindung bedeutet, oder
- eine Gruppe der Formel $-NR^{12}$ bedeutet,
worin
$R^{12}$ - für Wasserstoff oder Niederalkyl steht,
Z - eine Gruppe der Formel $-CH=CH-$ oder $-CH_2$-bedeutet, oder

- ein Schwefelatom, Sauerstoffatom oder
- eine Gruppe der Formel $-NR^{12}$ bedeutet,

worin

$R^{12}$ die oben angegebene Bedeutung hat,

und

n und m gleich oder verschieden sind und

- eine der aufgeführten Zahlen 0, 1, 2, 3 oder 4 bedeutet.

3. Substituierte annellierte Pyrrole nach den Ansprüchen 1 und 2

worin

$R^1$ - Pyridyl, Pyrimidyl, Chinolyl oder Isochinolyl bedeutet, das durch Fluor, Chlor, Methyl, Methoxy oder Trifluormethyl substituiert sein kann, oder

- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,

$R^2$ - Pyridyl, Pyrimidyl, Chinolyl oder Isochinolyl bedeutet, das durch Fluor, Chlor, Methyl, Methoxy oder Trifluormethyl substituiert sein kann, oder

- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl, oder
- Cyclopropyl, Cyclopentyl oder Cyclohexyl bedeutet, oder
- Methyl, Ethyl, Propyl, Isopropyl, Butyl, sec.-Butyl oder tert.Butyl bedeutet, das substituiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,

$R^3$, $R^4$, $R^5$ und $R^6$ gleich oder verschieden sind und

- Wasserstoff, Cyclopropyl, Cyclopentyl oder Cyclohexyl bedeuten, oder
- Methyl, Ethyl, Propyl, Isopropyl, Butyl, Isobutyl, tert.Butyl, Pentyl, Isopentyl, Hexyl oder Isohexyl bedeuten, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^7R^8$,

wobei

$R^7$ und $R^8$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,

oder durch Pyridyl, Pyrimidyl, Pyrazinyl, Pyridazinyl, Chinolin, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder

- Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl bedeuten, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
- Phenyl bedeuten, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy,

Isobutoxy, tert.-Butoxy, Methylthio, Ethylthio, Propylthio, Isopropylthio, Butylthio, Isobutylthio, tert.-Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^7R^8$ substituiert sein kann,

wobei

$R^7$ und $R^8$ die oben angegebene Bedeutung haben, oder

$R^3$ und $R^4$ und/oder $R^5$ und $R^6$ jeweils gemeinsam einen Ring bilden, der gegebenenfalls durch ein Sauerstoffatom, ein Schwefelatom oder durch eine Gruppe der Formel $-NR^9$ unterbrochen sein kann,

worin

$R^9$ - für Wasserstoff, Methyl, Ethyl, Proyl, Isopropyl, tert.Butyl, Phenyl, Benzyl, Methoxycarbonyl, Ethoxycarbonyl, Isopropoxycarbonyl, Propoxycarbonyl, Butoxycarbonyl, Isobutoxycarbonyl oder tert.Butoxycarbonyl steht,

X - eine Gruppe der Formel -CH=CH- bedeutet

A - eine Gruppe der Formel

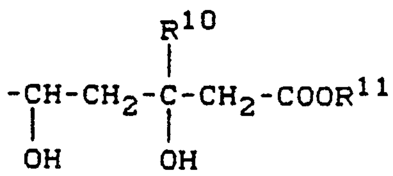

oder

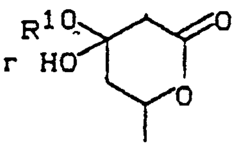

bedeutet,

worin

$R^{10}$ - für Wasserstoff, Methyl, Ethyl, Propyl, Isopropyl, Butyl, Isobutyl oder tert.Butyl steht,

$R^{11}$ - für Wasserstoff, Methyl, Ethyl, Propyl, Isopropyl, Butyl, Isobutyl, tert.Butyl oder Benzyl steht, oder

- für ein Natrium-, Kalium-, Calcium- oder Magnesium- oder Ammoniumion steht,

Y - eine Bindung bedeutet, oder

- eine Gruppe der Formel $-NR^{12}$ bedeutet,

worin

$R^{12}$ - für Wasserstoff, Methyl, Ethyl, Propyl, Isopropyl oder tert.Butyl steht,

Z - eine Gruppe der Formel -CH=CH- oder $-CH_2$-bedeutet, oder

- ein Schwefelatom, Sauerstoffatom oder

- eine Gruppe der Formel $-NR^{12}$ bedeutet,

worin

$R^{12}$ die oben angegebene Bedeutung hat,

und

m und n gleich oder verschieden sind und

- eine der aufgeführten Zahlen 0, 1, 2, 3 oder 4 bedeuten.

4. Substituierte anellierte Pyrrole nach den Ansprüchen 1 bis 3 zur therapeutischen Behandlung.

5. Verfahren zur Herstellung von substituierten anellierten Pyrrolen der allgemeinen Formel

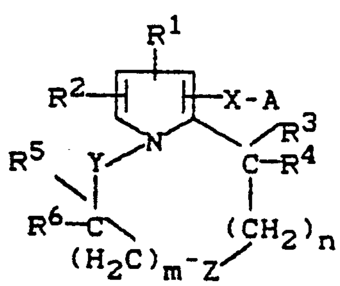

(I)

in welcher

$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin
$R^7$, $R^8$ - gleich oder verschieden sind und Alkyl, Aryl, Aralkyl, Acyl, Alkylsulfonyl oder Arylsulfonyl bedeuten,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,
$R^2$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,
worin
$R^7$, $R^8$ - die oben angegebene Bedeutung haben,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,
oder
- Cycloalkyl bedeutet, oder
- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,
worin
$R^7$, $R^8$ - die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^3$, $R^4$, $R^5$, $R^6$ gleich oder verschieden sind und
- Wasserstoff bedeuten, oder
- Cycloalkyl bedeuten, oder
- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,
worin
$R^7$, $R^8$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
- Heteroaryl bedeuten, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,
worin
$R^7$ und $R^8$ die oben angegebene Bedeutung haben,
oder
- Aryl bedeuten, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,

oder $R^3$ und $R^4$ und/oder $R^5$ und $R^6$ jeweils gemeinsam einen Ring bilden, der gegebenenfalls durch ein Sauerstoffatom, ein Schwefelatom, oder eine Gruppe der Formel $-NR^9$ unterbrochen sein kann,
worin
$R^9$ - für Wasserstoff, Alkyl, Aryl, Carbamoyl, Alkoxycarbonyl steht,
X - eine Gruppe der Formel $-CH_2-CH_2-$ oder $-CH=CH-$bedeutet,
A - eine Gruppe der Formel

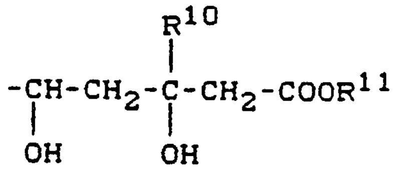

oder

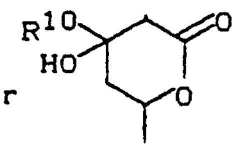

bedeutet,
worin
$R^{10}$ - für Wasserstoff oder Alkyl steht,
und
$R^{11}$ - für Wasserstoff steht, oder
- für Alkyl, Aralkyl oder Aryl steht, oder
- für ein Kation steht,
Y - eine Bindung bedeutet, oder
- eine Gruppe der Formel $-NR^{12}$ bedeutet
worin
$R^{12}$ - für Wasserstoff oder Alkyl steht,
Z - für eine Gruppe der Formel $-CH=CH-$ oder $-CH_2$ steht, oder
- für ein Schwefelatom, Sauerstoffatom, oder
- für eine Gruppe der Formel $-NR^{12}$ steht,
worin
$R^{12}$ die oben angegebene Bedeutung hat,
und
n und m gleich oder verschieden sind und
- eine Zahl 0, 1, 2, 3 oder 4 bedeuten,
dadurch gekennzeichnet, daß man
Ketone der allgemeinen Formel (VIII)

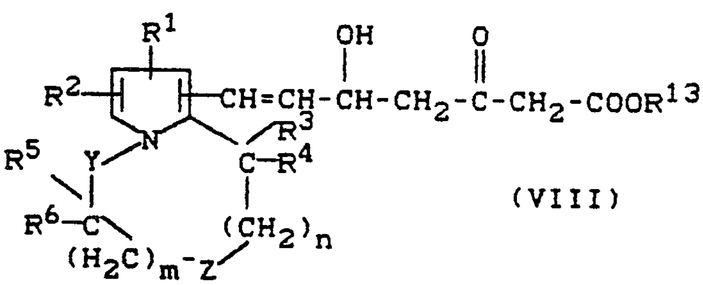

(VIII)

in welcher
$R^1$, $R^2$, $R^3$, $R^4$, $R^5$, $R^6$, Y, Z, m und n die oben angegebene Bedeutung haben
und
$R^{13}$ - für Alkyl steht,
reduziert,
und im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethanverbindung ($X = -CH_2-CH_2-$) die Ethenverbindung ($X = -CH=CH-$) nach üblichen Methoden hydriert,
und gegebenenfalls Isomere trennt.

6. Verfahren nach Anspruch 5, dadurch gekennzeichnet, daß die Reduktion im Temperaturbereich von

-80 bis +30° C durchgeführt wird.

7. Ketone der allgemeinen Formel (VIII)

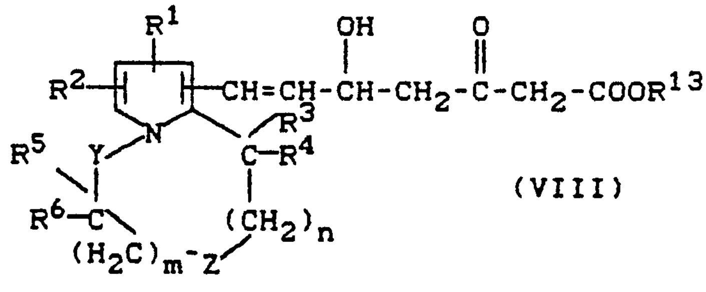

(VIII)

in welcher

$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$, $R^8$ - gleich oder verschieden sind und Alkyl, Aryl, Aralkyl, Acyl, Alkylsulfonyl oder Arylsulfonyl bedeuten,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben,

$R^2$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$, $R^8$ - die oben angegebene Bedeutung haben,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben,

oder

- Cycloalkyl bedeutet, oder

- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,

worin

$R^7$, $R^8$ - die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio, oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^3$, $R^4$, $R^5$, $R^6$ gleich oder verschieden sind und

- Wasserstoff bedeuten, oder

- Cycloalkyl bedeuten, oder

- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluomethylsulfonyl, Alkoxycarbonyl, Acyl oder durch eine Gruppe der Formel $-NR^7R^8$,

worin

$R^7$, $R^8$ die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

- Heteroaryl bedeuten, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$ und $R^8$ die oben angegebene Bedeutung haben,

oder

- Aryl bedeuten, das bis zu 5-fach gleich oder verschieden substituiert sein kann durch Alkyl, Alkoxy, Alkylthio, Alkylsulfonyl, Aryl, Aryloxy, Arylthio,

Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben,

oder $R^3$ und $R^4$ und/oder $R^5$ und $R^6$ jeweils gemeinsam einen Ring bilden, der gegebenenfalls durch ein Sauerstoffatom, ein Schwefelatom, oder eine Gruppe der Formel $-NR^9$ unterbrochen sein kann,

worin

$R^9$ - für Wasserstoff, Alkyl, Aryl, Carbamoyl, Alkoxycarbonyl steht,

Y - eine Bindung bedeutet, oder

- eine Gruppe der Formel $-NR^{12}$ bedeutet

worin

$R^{12}$ - für Wasserstoff oder Alkyl steht,

Z - eine Gruppe der Formel $-CH=CH-$ oder $-CH_2$ bedeutet, oder

- ein Schwefelatom, Sauerstoffatom, oder

- eine Gruppe der Formel $-NR^{12}$ bedeutet,

worin

$R^{12}$ die oben angegebene Bedeutung hat,

n und m gleich oder verschieden sind und

- eine Zahl 0, 1, 2, 3 oder 4 bedeuten,

und

$R^{13}$ - Alkyl bedeutet.

8. Verfahren zur Herstellung von ketonen der allgemeinen Formel

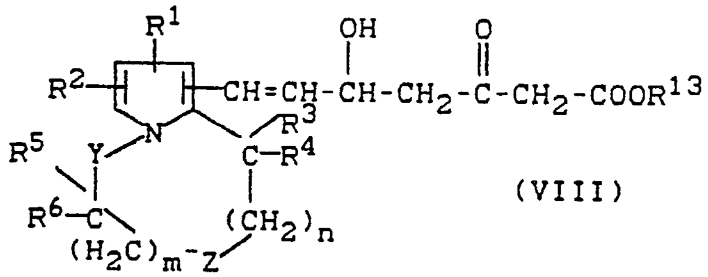

(VIII)

in welcher

$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$, $R^8$ - gleich oder verschieden sind und Alkyl, Aryl, Aralkyl, Acyl, Alkylsulfonyl oder Arylsulfonyl bedeuten,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,
$R^2$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,
worin
$R^7$ und $R^8$ - die oben angegebene Bedeutung haben,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,
oder
- Cycloalkyl bedeutet, oder
- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,
worin
$R^7$, $R^8$ - die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio, oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^3$, $R^4$, $R^5$, $R^6$ gleich oder verschieden sind und
- Wasserstoff bedeuten, oder
- Cycloalkyl bedeuten, oder
- Alkyl bedeuten, das substituiert sein kann durch Halogen, Cyano, Alkoxy, Alkylthio, Alkylsulfonyl, Trifluormethyl, Trifluor methoxy, Trifluormethylthio, Trifluomethylsulfonyl, Alkoxycarbonyl, Acyl oder durch eine Gruppe der Formel $-NR^7R^8$,
worin
$R^7$, $R^8$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
- Heteroaryl bedeuten, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,
worin
$R^7$ und $R^8$ die oben angegebene Bedeutung haben,
oder
- Aryl bedeuten, das bis zu 5-fach gleich oder verschieden substituiert sein kann durch Alkyl, Alkoxy, Alkylthio, Alkylsulfonyl, Aryl, Aryloxy, Arylthio,
Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,
oder $R^3$ und $R^4$ und/oder $R^5$ und $R^6$ jeweils gemeinsam einen Ring bilden, der gegebenenfalls durch ein Sauerstoffatom, ein Schwefelatom, oder eine Gruppe der Formel $-NR^9$ unterbrochen sein kann,
worin
$R^9$ - für Wasserstoff, Alkyl, Aryl, Carbamoyl, Alkoxycarbonyl steht,
Y - eine Bindung bedeutet, oder
- eine Gruppe der Formel $-NR^{12}$ bedeutet

worin
$R'^2$ - für Wasserstoff oder Alkyl steht,
Z - eine Gruppe der Formel -CH=CH- oder $-CH_2$ bedeutet, oder
- ein Schwefelatom, Sauerstoffatom, oder
- eine Gruppe der Formel $-NR'^{12}$ bedeutet,
worin
$R'^2$ die oben angegebene Bedeutung hat,
n und m gleich oder verschieden sind und
- für eine Zahl 0, 1, 2, 3 oder 4 stehen,
und
$R'^3$ Alkyl bedeutet,
dadurch gekennzeichnet, daß man
Aldehyde der allgemeinen Formel (IX)

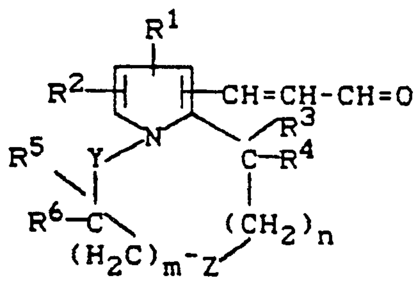
(IX)

in welcher
$R'$, $R^2$, $R^3$, $R^4$, $R^5$, $R^6$, Y, Z, m und n die oben angegebene Bedeutung haben,
in inerten Lösemitteln mit Acetessigestern der allgemeinen Formel (X)

$H_3O-\overset{\overset{O}{||}}{C}-CH_2-COOR'^{3}$ (X)

in welcher
$R'^3$ die oben angegebene Bedeutung hat,
in Anwesenheit von Basen umsetzt.

9. Aldehyde der allgemeinen Formel

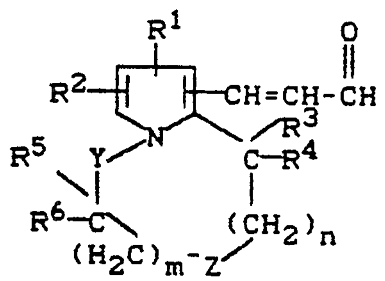
(IX)

in welcher
$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,
worin
$R^7$, $R^8$ - gleich oder verschieden sind und Alkyl, Aryl, Aralkyl, Acyl, Alkylsulfonyl oder Arylsulfonyl bedeuten,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin

$R^7$ und $R^8$ die oben angegebene Bedeutung haben,

$R^2$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$, $R^8$ - die oben angegebene Bedeutung haben,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben,

oder

- Cycloalkyl bedeutet, oder

- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,

worin

$R^7$, $R^8$ - die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio, oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^3$, $R^4$, $R^5$, $R^6$ gleich oder verschieden sind und

- Wasserstoff bedeuten, oder

- Cycloalkyl bedeuten, oder

- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluomethylsulfonyl, Alkoxycarbonyl, Acyl oder durch eine Gruppe der Formel $-NR^7R^8$,

worin

$R^7$, $R^8$ die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

- Heteroaryl bedeuten, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$ und $R^8$ die oben angegebene Bedeutung haben,

oder

- Aryl bedeuten, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben,

oder $R^3$ und $R^4$ und/oder $R^5$ und $R^6$ jeweils gemeinsam einen Ring bilden, der gegebenenfalls durch ein Sauerstoffatom, ein Schwefelatom, oder eine Gruppe der Formel $-NR^9$ unterbrochen sein kann,

worin

$R^9$ - für Wasserstoff, Alkyl, Aryl, Carbamoyl, oder Alkoxycarbonyl steht,

Y - eine Bindung bedeutet, oder

- eine Gruppe der Formel $-NR^{12}$ bedeutet

worin

$R^{12}$ - für Wasserstoff oder Alkyl steht,

Z - eine Gruppe der Formel $-CH=CH-$ oder $-CH_2$ bedeutet, oder

- ein Schwefelatom, Sauerstoffatom, oder
- eine Gruppe der Formel $-NR^{12}$ bedeutet,
worin
$R^{12}$ die oben angegebene Bedeutung hat,
und
n und m gleich oder verschieden sind und
- für eine Zahl 0, 1, 2, 3 oder 4 stehen.

10. Verfahren zur Herstellung von Aldehyden der allgemeinen Formel

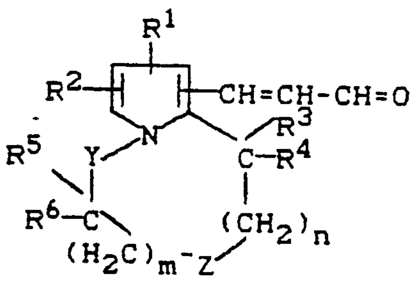

(IX)

in welcher
$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,
worin
$R^7$, $R^8$ - gleich oder verschieden sind und Alkyl, Aryl, Aralkyl, Acyl, Alkylsulfonyl oder Arylsulfonyl bedeuten,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,
$R^2$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,
worin
$R^7$, $R^8$ - die oben angegebene Bedeutung haben,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,
worin
$R^7$ und $R^8$ die oben angegebene Bedeutung haben,
oder
- Cycloalkyl bedeutet, oder
- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^7R^8$,
worin
$R^7$, $R^8$ - die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio, oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^3$, $R^4$, $R^5$, $R^6$ gleich oder verschieden sind und
- Wasserstoff bedeuten, oder

- Cycloalkyl bedeuten, oder

- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluomethylsulfonyl, Alkoxycarbonyl, Acyl oder durch eine Gruppe der Formel $-NR^7R^8$,

worin

$R^7$, $R^8$ die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

- Heteroaryl bedeuten, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^7R^8$ substituiert sein kann,

worin

$R^7$ und $R^8$ die oben angegebene Bedeutung haben,

oder

- Aryl bedeuten, das bis zu 5-fach gleich oder verschieden substituiert sein kann durch Alkyl, Alkoxy, Alkylthio, Alkylsulfonyl, Aryl, Aryl oxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^7R^8$,

worin

$R^7$ und $R^8$ die oben angegebene Bedeutung haben,

oder $R^3$ und $R^4$ und/oder $R^5$ und $R^6$ jeweils gemeinsam einen Ring bilden, der gegebenenfalls durch ein Sauerstoffatom, ein Schwefelatom, oder eine Gruppe der Formel $-NR^9$ unterbrochen sein kann,

worin

$R^9$ - für Wasserstoff, Alkyl, Aryl, Carbamoyl, oder Alkoxycarbonyl steht,

Y - eine Bindung bedeutet, oder

- eine Gruppe der Formel $-NR^{12}$ bedeutet

worin

$R^{12}$ - für Wasserstoff oder Alkyl steht,

Z - eine Gruppe der Formel $-CH=CH-$ oder $-CH_2$ bedeutet, oder

- ein Schwefelatom, Sauerstoffatom, oder

- eine Gruppe der Formel $-NR^{12}$ bedeutet,

worin

$R^{12}$ die oben angegebene Bedeutung hat,

und

n und m gleich oder verschieden sind und

- eine Zahl 0, 1, 2, 3 oder 4 bedeuten,

dadurch gekennzeichnet, daß man

substituierte Pyrrole der allgemeinen Formel (XI)

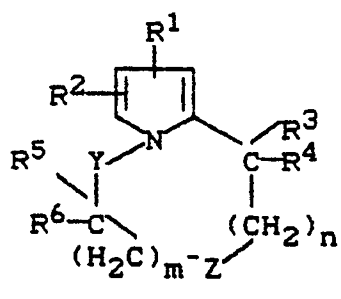

(XI)

in welcher

$R^1$, $R^2$, $R^3$, $R^4$, $R^5$, $R^6$, Y, Z, m und n die oben angegebene Bedeutung haben,

in inerten Lösemitteln in Anwesenheit von Hilfsstoffen mit N,N-Dialkylaminoacrolin der Formel (XII)

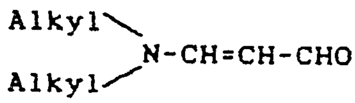 (XII)

wobei

Alkyl - für einen geradkettigen oder verzweigten Kohlenwasserstoffrest mit 1 bis 6 Kohlenstoffatomen steht, umsetzt.

11. Arzneimittel, enthalten substituierte annellierte Pyrrole nach den Ansprüchen 1 bis 3.

12. Arzneimittel nach Anspruch 11, dadurch gekennzeichnet, daß es 0,5 bis 98 Gew.-% substituierte annellierte Pyrrole bezogen auf die Gesamtmischung enthält.

13. Verwendung von substituierten annellierten Pyrrolen nach den Ansprüchen 1 bis 3 zur Behandlung von Krankheiten.

14. Verwendung von substituierten anellierten Pyrrolen nach den Ansprüchen 1 bis 3 zur Herstellung von Arzneimitteln.

15. Verwendung nach Anspruch 14 zur Herstellung von Inhibitoren der 3-Hydroxy-3-methyl-glutaryl-Coenzym A (HMG-CoA) Reduktase.